# EUROPEAN PATENT SPECIFICATION

(11) **EP 3 942 570 B1**
(45) Date of publication and mention of the grant of the patent: **12.08.2026**
(21) Application number: 19725213.3
(22) Date of filing: 21.03.2019
(51) Int. Cl.: G16H 20/10, G16H 10/60, G16H 70/40, G16H 50/20

(54) **SYSTEM, METHOD AND SOFTWARE PROGRAM FOR MANAGING THE INTERACTION BETWEEN DRUGS**
SYSTEM, VERFAHREN UND SOFTWAREPROGRAMM ZUR VERWALTUNG DER INTERAKTION ZWISCHEN MEDIKAMENTEN
SYSTÈME, PROCÉDÉ ET LOGICIEL POUR GÉRER LES INTERACTIONS ENTRE DES MÉDICAMENTS

(43) Date of publication of application: 26.01.2022
(73) Proprietor: DRUG-PIN S.r.l., 20135 Milan (IT)
(72) Inventor: SIMMACO, Mario Edoardo, 00189 Roma (IT); PREISSNER, Sally, 10407 Berlin (DE)
(74) Representative: PGA S.p.A., Milano, Succursale di Lugano
(86) International application number: PCT/IB2019/052310
(87) International publication number: WO 2020/188322

(56) References cited:
- US-A1- 2009 094 059
- US-A1- 2016 004 838
- ANONYMOUS: "Enzyme inhibitor - Wikipedia", 15 March 2019 (2019-03-15), XP055643209, Retrieved from the Internet <URL:https://en.wikipedia.org/w/index.php?title=Enzyme_inhibitor&oldid=887936210> [retrieved on 20191115]

## Description

### Field of the invention

The present invention relates to a system for managing and calculating the interaction between drugs.

The present invention also relates to a method for managing and calculating the interaction between drugs.

Finally, the present invention relates to a software program for managing and calculating the interaction between drugs.

### Prior art

Over the past few years, pharmaceutical research has allowed the development of a large number of drugs for the treatment of many diseases. Each drug is typically aimed at treating (eradicating) or slowing the evolution of a specific disease or reducing the associated symptoms, despite the presence of a different number of side effects, varying in number and intensity in each individual subject.

However, in addition to the side effects of a single drug, widely described in the data sheets and in the illustrative leaflets of the individual products, patients taking multiple drugs may suffer negative effects as a result of their interactions or the body's different capacity to metabolize (i.e. eliminate) the drugs, with consequent modifications of their effectiveness and/or of the onset of further serious side effects. Although it is mandatory to report in the data sheet of a single drug its known interactions with other active ingredients or with food, it is very complex and difficult to apply in practice to analyze the interactions between many drugs.

In fact, the risk of a negative drug interaction increases with the number of diseases present in the individual and, consequently, with the number of drugs necessary to treat each disease. For example, some anti-inflammatory drugs, used for analgesic purposes, may have negative influences on kidney function or blood pressure and should be used with great caution in patients who already have high blood pressure or kidney disease. Finally, while interactions between two drugs are known and generally carefully evaluated, the interactions between multiple drugs are much more complex and poorly assessable.

A "polytreated" patient is a patient who continuously takes several drugs at the same time following the presence of various diseases, each of which requires a specific pharmacological treatment. The World Health Organization (WHO) considers patients who take at least 5 different drugs a day as "polytreated".

Although the regulatory bodies (FDA, EMA, AIFA, to name but a few) have indicated specific clinical pathways to evaluate negative drug interactions in patients treated for the simultaneous presence of different diseases, there are still no simple methods of clinical use for a holistic evaluation of these patients *(*Loscalzo J, Barabasi A-L. Systems biology and the future of medicine. WIREs Syst Biol Med2011; 3: 619-27.), with significant consequences in terms of avoidable costs and suffering for the National Health System and for patients.

Elderly patients are obviously those most exposed to these risks, not only because they are more likely to be subjected to chronic diseases (for example, diabetes, obesity, hypertension, osteoarticular diseases) which require different pharmacological treatment (making them "polytreated" according to the definition of the WHO), but also because the metabolic capacities of the organism change with age, with consequent changes in the pharmacokinetics and pharmacodynamics of the different compounds taken, followed by further side effects. A study by Morgan, T.K., et al. ("A national census of medicines use: a 24-hour snapshot of Australians aged 50 years and older." Med J Aust, 2012, 196, 50-53*)* showed that about 45% of people over 50 regularly take more than 5 different types of drugs. In Italy, Onder et al. (Prescription Drug Use Among Older Adults in Italy: A Country-Wide Perspective, JAMDA 2014: 15,7: 531-535*)* have documented that of over 12 million over sixty-five patients there are more than 7 million taking 5 to over 10 drugs a day.

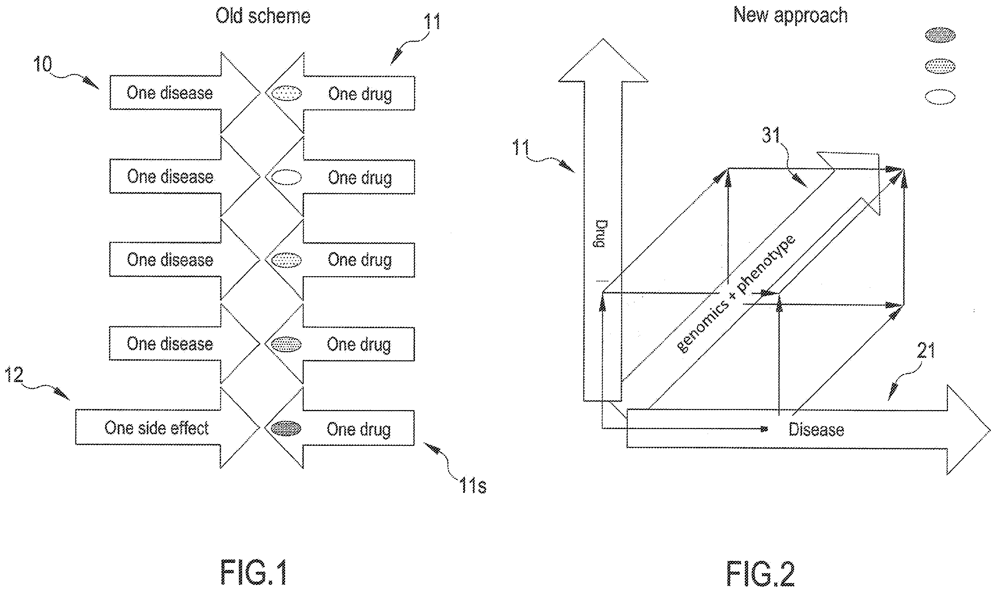

According to a traditional approach, schematically illustrated in figure 1, for each disease 10 a drug 11 is proposed which is able to cure that specific disease. Figure 1 illustrates an exemplary condition in which four distinct diseases 10 are present, and therefore, for each of these a drug 11 is proposed, for a total of 4 treatment drugs only for the respective diseases.

The intake of various drugs, of heterogeneous nature, can lead to interactions: a drug can be combined, in a positive or negative manner, with other drugs. With the increase in the number of drugs taken by an individual, the management of drug interaction becomes more and more critical, because with the increasing number of drugs taken simultaneously, the risk of even significant negative interactions increases significantly. Some interactions between drugs can in fact lead to the death of the individual who takes them.

Again in figure 1, the presence of a side effect 12 is often cured by administering a further drug, indicated in figure 1 with the reference numeral 11s. In this context of care, it is often difficult to understand whether a given symptom derives from the failure to control one of the diseases affecting the patient or from a negative interaction between the drugs, from known incompatibilities or changes in their metabolism due to alterations of a single subject.

Negative interactions that can develop between drugs taken at the same time can lengthen the treatment time of one or more diseases, affect the effects of other drugs to the point that they are useless for the treatment of the disease for which they were originally intended, and can lead to hospitalization of the subject taking these drugs, or even to the onset of new diseases. All this entails a reduction in the overall state of health of the individual and in many cases a useless expenditure.

Some databases are known, including "Transformer", available at the site http://bioinformatics.charite.de/transformer, which contains - at the time of writing the present application - data of about 2800 drugs, with about 60,000 indications of possible drug conflicts, of which 5,500 interactions relating to phase I metabolism (cytochrome P450 or CYP enzymes, which represents the body's main detoxification mechanism from drugs and which is one of the factors that most contribute to define the variability of the dose/response ratio in different subjects taking the same drug) and/or phase II (other enzymes), and which also includes data on pharmacokinetic transporters, food interactions (about 350) and over 100,000 bibliographic references.

The analysis of the above database is very complicated and today it is not an optimal means for the management of drug interactions.

As already mentioned above, in fact, various factors including the ethnicity of the subject 200 and the type of drug affect the occurrence or not of interactions on the combination of different drugs that the subject takes. For example, drugs such as benzodiazepines or other psychoactive drugs can produce adverse interactions depending on the dose at which they are taken.

Some programs aimed at evaluating drug interactions are available on the Web (Drug Interaction Checker: www.webmd.com/interaction-checker/default.htm, www.rxlist.com/drug-interaction-checker.htm, https://reference.medscape.com/drug-interactionchecker, www.drugs.com/drug_interactions.html).

However, these programs are limited to identifying possible drug interactions with another present in the list entered by the doctor, providing a long list of possible negative interactions. After reading the entire sequence of interactions, the physician must reformulate the prescription, subjecting it to the system again in an iteration that can take a very long time, incompatible with clinical needs. Finally, in these programs the clinical characteristics of a single subject or his/her concomitant diseases capable of modifying the drug's bioavailability are never evaluated.

US 2009/0094059A1 relates to a computer-based platform that integrates genetic, pharmacologic and clinical information in a central database and applies predictive algorithms to generate personalized drug-gene, drug-drug and drug-substance interaction reports via a graphical user interface.

US 2016/0004838 A1 discloses methods and systems for improving predictions of drug interactions, such as through comparison with clinical data, and treating based on the improved predictions. An area under the curve (AUC) for substance-factor interactions is predicted, correction factors are applied and improved prediction of change in AUC for substance-factor interactions is calculated after applying correction factors.

The object of the present disclosure is to describe a method, a system and a software program for managing and calculating the interaction between drugs which allow an optimal analysis of the interactions and which allow in an effective and rapid manner to determine the best combination or cocktail of drugs to be assigned to a subject suffering simultaneously from multiple diseases, for which protracted treatments are necessary over time.

### Summary

The object of the present invention is described in relation to one or more aspects which can be combined with each other and/or with portions of the following detailed description and/or with the appended claims.

According to a first aspect, a method for managing and calculating the interaction between according to claim 1 is disclosed.

According to a further non-limiting aspect, said introduction of electronic data (101) comprises the introduction of said data (101) in an electronic computer, and comprises the saving, in particular at least temporarily, of said electronic data (101).

According to a further non-limiting aspect, the cocktail (100) of drugs comprises over-the-counter drugs and/or drugs subject to medical prescription, and/or supplements and/or vitamins and/or phytopharmaceuticals and/or herbs.

According to a further non-limiting aspect, the optimization research step of said score comprises the proposition of a combination of drugs distinct with respect to said cocktail (100) and/or with respect to said drugs forming said cocktail (100). According to a further non-limiting aspect, said combination of drugs comprises a plurality of drugs of which at least one is a substitute drug, different from the drugs of said cocktail (100) and/or which contributes or makes said combination of drugs different from said cocktail (100), and/or said combination of drugs comprises a smaller number of drugs than the number of drugs forming part of said cocktail (100).

According to a further non-limiting aspect, said combination of drugs comprises over-the-counter drugs and/or drugs subject to medical prescription, and/or supplements and/or vitamins and/or phytopharmaceuticals and/or herbs.

According to a further non-limiting aspect, said optimization research step of the score is an electronic optimization step, optionally automatically performed by a software program and/or electronically and/or automatically performed by an electronic computer.

According to a further non-limiting aspect, said research optimization step of said score includes the electronic calculation of a first score based on the drugs of said cocktail (100) and an electronic calculation of a second score based on the drugs of said combination of drugs.

According to a further non-limiting aspect, the method comprises a step of presenting an electronic interface for performing said optimization step, optionally wherein at least the first score and the second score are displayed alternately or in combination.

According to a further non-limiting aspect, said first plurality of sum factors and said second plurality of sum factors, and/or said calculation step take into account the genotype of said subject (200).

According to a further non-limiting aspect, at least said calculation step, and/or said score optimization step, are performed by electronic access to a drug database (304) and/or following access from a drug database (304), in particular from which interaction data and/or data related to side effects are taken.

According to a further non-limiting aspect, said drug database (304) is a drug database comprising non-commercial names and/or active ingredients of drugs.

According to a further non-limiting aspect, said summation is performed for a predefined number (E) of enzymes (c), optionally for at least a part and/or for a plurality of enzymes, preferably all enzymes, involved in the metabolization of at least one drug, and preferably of all the drugs, of said cocktail (100).

According to a further non-limiting aspect, said subject (200) is an individual suffering from one or more diseases, in particular treated with said cocktail (100).

According to a further non-limiting aspect, the method comprises, following the introduction of said habit data (48), a step of electronic search of data for inhibiting and/or affecting enzymes in association with said habit data, in particular enzymes affected or inhibited by the use of alcohol and/or caffeine and/or smoke; said calculation step being performed by a correction of the score induced and/or generated by said enzyme inhibition and/or affection data.

The second plurality of summation factors is calculated on each drug (d) of drugs of said cocktail (100) of drugs taken by the subject (200).

According to a further non-limiting aspect, there is a step of changing at least one drug (d) among the drugs forming part of the cocktail (100), in particular for the definition of an at least temporary new cocktail of drugs.

According to a further non-limiting aspect, there is a new step of electronic calculation of said score, performed following the step of changing said at least one drug (d) and/or performed on said new cocktail (100) of drugs.

According to a further non-limiting aspect, the new electronic calculation step of said score leads to the calculation of said second score.

According to a further non-limiting aspect, the step of changing said at least one drug (d) is performed automatically, optionally being performed by at least one electronic computer and/or data processing unit.

According to a further non-limiting aspect, the step of changing said at least one drug (d) is performed manually by selecting a specific drug alternative to said drug (d) present in the cocktail (100).

According to a further non-limiting aspect, the step of changing at least one drug (d) among the drugs forming part of the cocktail (100) leads to the creation of said combination of drugs.

According to a further non-limiting aspect, said personal parameters and/or blood values (45) comprise data, and/or are indicative of intestinal function of said subject (200).

According to a further non-limiting aspect, the method comprises a step of defining a sub-sector or subset (S) of drugs, which are part of the cocktail (100) of drugs taken by the subject (200), said sub-sector or subset (S) of drugs including drugs considered essential and/or not modifiable; the step of changing said at least one drug being performed on at least one drug of said cocktail (100) not forming part of said sub-sector or subset (S), being in particular performed on all the drugs of said cocktail (100) not forming part of said sub-sector or subset (S) and/or being performed excluding the drugs of said sub-sector or subset (S) from said change.

According to a further non-limiting aspect, there is a step of updating said database, said updating step being optionally and automatically performed at predetermined time intervals.

According to a further non-limiting aspect, at least the calculation of said first plurality of sum factors and of the second plurality of sum factors is performed in relation to non-commercial names and/or active ingredients of drugs stored in said drug database (304), and/or is performed taking into account the interaction data and/or related to side effects contained in said drug database (304), in particular concerning at least one drug (d) of the cocktail (100).

According to a further non-limiting aspect, the method comprises a step of electronic access to a database of alarm and/or warning factors (S_{d}, W_{d}), and comprises, for each drug (d), and/or for each d-th drug, of the cocktail (100) of drugs taken by the subject (200), an electronic research step performed automatically for the search of said alarm and/or warning factors (S_{d}, W_{d}).

According to a further non-limiting aspect, said database of alarm and/or warning factors is accessible from and/or included in the drug database (304).

According to a further non-limiting aspect, said alarm and/or warning factors (S_{d}, W_{d}) comprise data and/or legal rules retrieved from technical sheets and/or illustrative leaflets and/or medical information intended for professionals of the health sector, said data including fatal cases due to interactions between drugs and/or due to combinations between drugs.

In the second plurality of sum factors, the alarm and/or warning factors (S_{d}, W_{d}), the corrective factor deriving from the action of prodrugs (Pro_{d}), and the physiological corrective factor (Pharm_{d}) relating to physiological data of said subject (200) are added together, and the second plurality of sum factors comprises the sum of at least one alarm and/or warning factor (S_{d}, W_{d}), of the correction factor deriving from the action of prodrugs (Pro_{d}), and of the physiological correction factor (Pharm_{d}) for each d-th drug of the cocktail (100) of drugs taken by the subject (200).

According to a further non-limiting aspect, said alarm and/or warning factors contribute as a factor with priority score in said second plurality of sum factors, and/or for each drug (d) of said cocktail (100) of drugs, the score relative to the alarm and/or warning factor (S_{d}, W_{d}), relative to said drug, is greater and/or preponderant with respect to the score relative to the correction factor deriving from prodrugs (Pro_{d}), and/or the physiological correction factor (Pharm_{d}).

According to a further non-limiting aspect, the correction factor deriving from the action of prodrugs (Pro_{d}) has a greater weight than the physiological correction factor (Pharm_{d}).

According to a further non-limiting aspect, the method comprises a step of emitting an alarm signal or sign when, for a predetermined d-th drug of said cocktail (100), there are alarm and/or warning factors (S_{d}, W_{d}) with a score different from a predetermined value, optionally different from zero.

According to a further non-limiting aspect, the step of identifying said cocktail (100) of drugs comprises identifying and storing the commercial and/or non-commercial name and/or the active ingredient of one or more drugs, optionally further comprising the storage of the doses taken for each drug of said cocktail (100) of drugs and/or the duration of administration of each drug of said cocktail (100) of drugs.

According to a further non-limiting aspect, said first plurality of sum factors is calculated on at least a predefined number of enzymes (c), optionally on all the enzymes (E), active in the metabolization of drugs, and is calculated taking into account at least the cytochrome P450.

The interaction factors between drugs include the total number of interactions (TIₑ) of the drugs forming part of said cocktail (100).

According to a further non-limiting aspect, drugs in said drug database (304) are classified according to the ATC (Anatomical Therapeutic Classification system) coding.

According to a further non-limiting aspect, said drug database (304) comprises drugs of the category of supplements and/or vitamins and/or phytopharmaceuticals and/or herbs, and/or is connected with one or more databases comprising drug data of the category of supplements and/or vitamins and/or phytopharmaceuticals and/or herbs.

For the purposes of this disclosure, "prodrug" means a nonpharmacologically active preparation, in particular at the time of administration to a subject (200), but which becomes so as a result of the metabolic processes undergone following the administration to said subject (200).

According to a further non-limiting aspect, said value of said score corresponding to the minimum possible adverse interaction is a minimum value of a curve defined on at least two dimensions and/or of a multidimensional curve when increasing scores are indicative of increasing adverse interactions, and is a maximum value of a curve defined on at least two dimensions and/or of a multidimensional curve when decreasing scores are indicative of increasing adverse interactions.

In said first plurality of sum factors, said corrective factor (M_{c,e}) extracted from the matrix (M) of corrective factors is multiplied by the factors of drug interactions (TIₑ, Iₑ).

According to a further non-limiting aspect, said drug interaction factors include the total number (TIₑ) of interactions between all the drugs taken by said subject (200) and all the enzymes (e) and the number of type C interactions (Iₑ) for all the drugs of said cocktail (100) and all the enzymes (e).

According to a further non-limiting aspect, the total number (TIₑ) of interactions between all the drugs taken by said subject (200) and all the enzymes (e) and the number of type C interactions (Iₑ) for all the drugs taken by said subject (200) and all the enzymes (e) are multiplied together to contribute to define said factor of the first plurality of sum factors.

According to a further non-limiting aspect, the total number (TIₑ) of interactions among all the drugs taken by said subject (200) and all the enzymes (e) and the number of C-type interactions (I) for all the drugs taken by said subject (200) and all the enzymes (e), is multiplied by said corrective factor (M_{c,e}) deriving from said matrix (M) of values, each comprising an interaction factor between a predefined drug and a predefined enzyme.

According to a further non-limiting aspect, the personal parameters and/or blood values (46) comprise at least part of the following data, and preferably all the following data: date of birth, age, gender, ethnicity, height, weight, body mass index, creatinine, GRF, bilirubin, gamma-glutamyl transpeptidase (GGT), alkaline phosphatase (or ALP), ALT, AST.

According to a further non-limiting aspect, the personal parameters also comprise data relating to the lifestyle of said subject (200) and/or the number of weekly hours devoted to exercise and/or exposure to the work environment and/or diet data including at least the indication of an omnivorous, vegetarian or vegan diet and/or the number and distribution of meals during the day, and/or comorbidity data.

According to a further non-limiting aspect, an access step is present to access a drug database (304) and/or to extract interaction data between drugs obtained from total pharmaceutical data comprising at least one, and preferably all, the following data:
- official provisions,
- Phase I and II metabolic enzymes (inhibitor drugs, inducing drugs, substrate drugs),
- transporters,
- molecular targets,
- clearance half-life, extra renal secreted fraction.

According to a further non-limiting aspect, said total pharmaceutical data comprise data relating to the concentration of drugs in the various biological liquids, according to the therapeutic monitoring of the drugs.

According to a further non-limiting aspect, the calculation step of said score includes a prior analysis and/or consideration of clinical laboratory data, said clinical laboratory data comprising at least one of the data of the following list:
- hormonal profile;
- intestinal permeability;
- hematological profile;
- coagulation profile;
- functional biological markers of endogenous and exogenous homeostasis,
and said clinical laboratory data contribute to the determination and/or alteration of said score.

There is further a research and electronic proposition step of at least one drug as an alternative to one or more drugs of said drug cocktail (100); said electronic proposition being calculated by automatically searching for a d-th drug of said cocktail (100), a drug whose active ingredient is of the same pharmacological class as the d-th drug and/or serves for treating the same disease.

According to a further non-limiting aspect, there is a step of selecting said drug as an alternative to one or more drugs of said drug cocktail (100), in particular to lead to the creation of said combination of drugs, following which the calculation step is preferably performed again.

According to a further non-limiting aspect, as a result of said selection step, said drug as an alternative to one or more drugs of said cocktail (100) is added to and/or becomes part of said composition of drugs alternative to said cocktail (100), becoming said substitute drug.

According to a further non-limiting aspect, the method comprises an electronic presentation step, on a user interface, of at least one tab (43) relating to single nucleotide polymorphisms (SNP) for said subject (200), in which data are presented relating to one or more enzymes (e), and optionally, for each enzyme (e) of said plurality of enzymes, there are data relating to the allele of the enzyme, to a nucleotide variation thereof and/or to a change in the activity associated with the enzyme or of the enzyme.

According to a further non-limiting aspect, the method comprises an electronic calculation step of the variation of the activity of one or more enzymes (e) of said plurality of enzymes, said variation being calculated based on, and/or being generated by and or because of one or more drugs (d) of said cocktail (100).

According to a further aspect, a software program is disclosed, comprising code portions which, when executed by a data processing unit, cause the execution of the steps according to one or more of the present aspects.

According to a further aspect, a memory support is disclosed, comprising said software program.

According to a further non-limiting aspect, said software program is configured to be usable remotely, for example by means of a web browser.

According to a further aspect, a system (300) for managing interactions between drugs is disclosed, comprising at least a data processing unit and a memory (304), on which a software program for the management of drug interactions is loaded, said data processing unit being configured to execute said software program which, when executed, executes the method according to one or more of the present aspects.

According to a further non-limiting aspect, said system (300) for managing interactions between drugs comprises at least one access interface to allow simultaneous access to the use of said software program, optionally by a plurality of users simultaneously.

According to a further non-limiting aspect, said system for managing interactions between drugs also comprises user interface means configured to allow the loading of data of one or more patients and to allow the display of a plurality of result data generated by said software program.

According to a further non-limiting aspect, a distributed computing environment is disclosed for managing interactions between drugs, comprising at least a data processing unit and a memory (304), on which a software program is loaded, comprising code portions that when executed cause the execution of the steps of the method according to one or more of the present aspects.

According to a further aspect, the use of the system (300) and/or of the distributed computing environment is described and/or the use of the method is described according to one or more of the preceding aspects for the reduction of adverse interactions in a drug cocktail (100), optionally a drug cocktail (100) for a polytreated patient.

### Description of the figures

The following detailed description relate to one or more non-limiting embodiments of the object of the present disclosure, described in relation to the accompanying figures in which:
- figure 1 illustrates a scheme of principle of action towards multiple diseases for a given subject, according to a known scheme;
- figure 2 illustrates a scheme of principle of action towards multiple diseases for a given subject, according to a scheme according to the present invention;
- figure 3 illustrates a scheme of principle of the method for managing interactions between drugs object of the present disclosure;

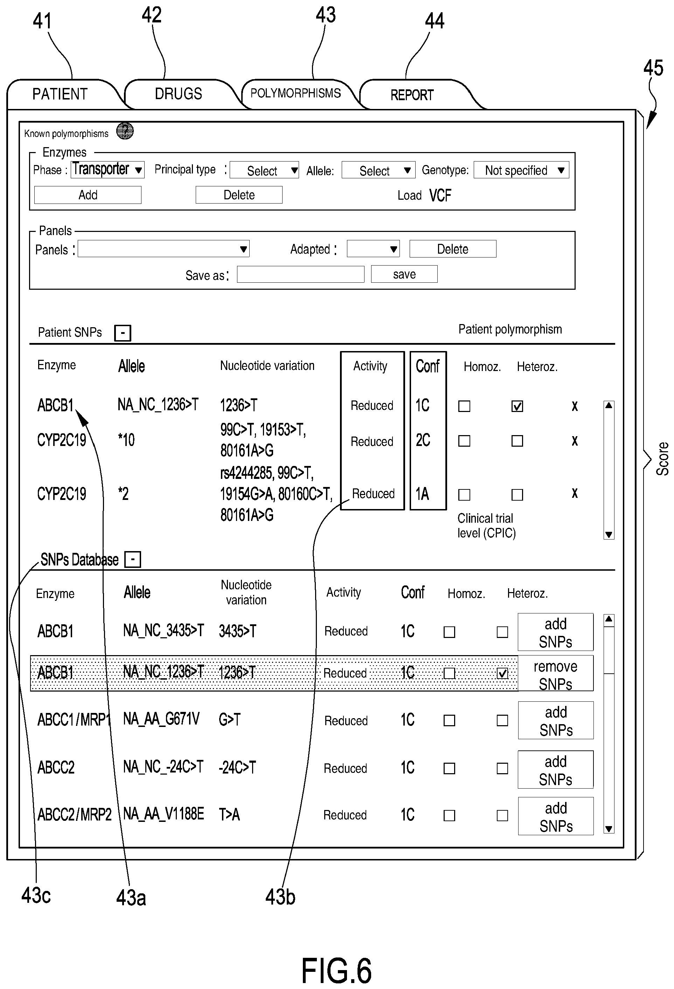

- figure 4, figure 5 and figure 6 illustrate a first, a second and a third screen, respectively, of a software program for managing the interaction between drugs according to the present invention;
- figure 7 illustrates a scheme of distribution of scores deriving from drug interaction calculated on the basis of a predefined sample of patients;
- figure 8 illustrates graphs relating to distributions of scores and frequencies for certain groups;
- figure 9 illustrates a graph relating to scores calculated on the basis of a plurality of four drugs;
- figure 10, figure 11 and figure 12 illustrate a fourth, a fifth and a sixth screen of said software program, in which the scores of the interaction of the drugs are subsequently modified.

### Detailed description

The approach to the management of the interaction between drugs according to the present disclosure follows a multidimensional scheme schematically represented in figure 2, in which medicaments 11 and diseases 21 are correlated in relation to genomics and phenotype aspects 31, and no longer in a 1:1 approach as generally used by the programs currently in use.

The present disclosure first of all relates to a method for managing the interaction between drugs, implemented by means of a specific computer program. Therefore in the following portion of description specific reference will be made to such a program.

In particular, the method described herein makes use of a software program for managing drug interactions, described herein in detail. According to the present disclosure, the set of drugs taken by a subject 200 or patient is defined as a cocktail, or more precisely a "drug cocktail". Although a drug cocktail may include traditional over-the-counter and/or medical prescription drugs, preferably, although not limited to, the drug cocktail includes data on supplements and vitamins taken by the subject 200, phytopharmaceuticals and herbs. Due to this aspect, the software program object of the present disclosure allows carrying out optimal analyzes on a wide spectrum, since also the influence of "non-traditional" drugs is contemplated. The Applicant has indeed observed that even supplements, vitamins, phytopharmaceuticals and herbs can contribute to determining interactions with other drugs, and therefore the analysis produced by the program is more complete if these product categories are also considered.

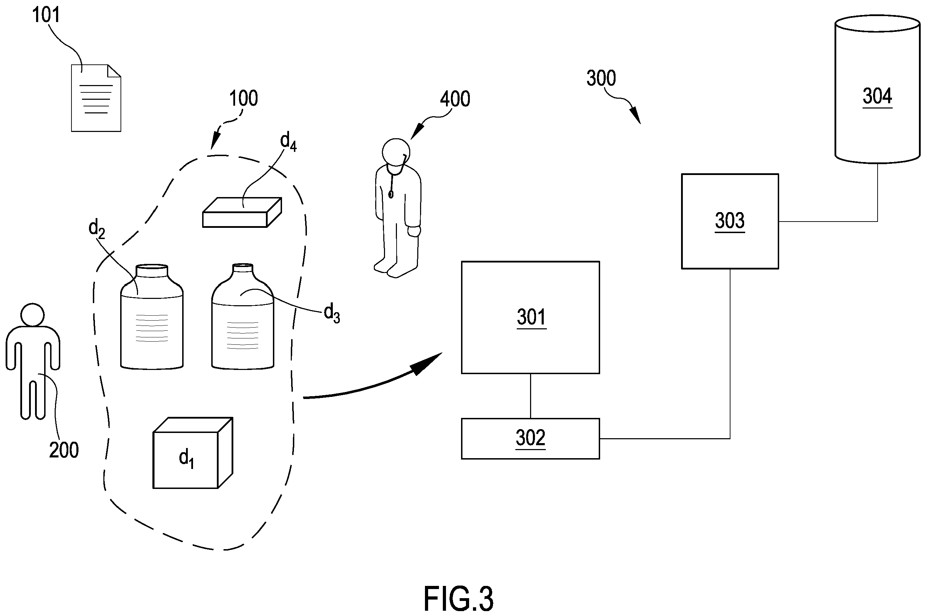

As shown in figure 3, the software program is for example executed on a server 303 of a system 300 for the analysis of drug interactions, which comprises a database 304, and which is preferably connected to and/or comprises a local unit 302 provided with user interface means 301 which allow a user 400 (for example the health professional) and/or the patient 200, to enter data (schematically indicated in figure 3 with the reference numeral 101) relating to personal parameters and/or blood values and/or current or past diseases of said patient 200, as well as the drugs of cocktail 100. The database 304 is preferably accessible by a plurality of users simultaneously, and the data included therein cannot be modified by the common users accessing the software. In particular, the names of drugs, in particular the non-commercial names of drugs and/or their active ingredients, are not modifiable by the user, and for this reason are described herein as "read-only". Preferably, although not limited to, all the data necessary for the operation of the software object of the present disclosure are contained in a single database; this makes it easier to manage the entire data set.

The cocktail 100 comprises a predetermined total number D of drugs, and each d-th drug is in figure 3 represented by the wording d1, d2, d3, d4. For the purposes of the present disclosure, the terms drug, medicine or medicament are to be understood as synonyms. Through the software program described herein, a set of parameters is defined for each patient according to the following description, and in particular through a genotype characterization of the specific subject 200 considered, a score is defined and is indicative of the level of interaction, in particular of adverse or negative interaction, between the drugs of the cocktail 100 taken by the patient. The purpose is to bring the score to a value corresponding to the lowest possible negative interaction. For this reason, the software program described herein has a user interface, through which it is possible to perform all the operations described hereinafter and which allows proposing changes in the drug cocktail 100, in particular changes including alteration and/or change of at least one drug among the drugs belonging to said cocktail 100, so as to make a "combination of drugs" different from the cocktail 100; a new score is calculated on this combination of drugs, which preferably is compared with the previous one to verify if the change of the at least one drug has brought benefits or not in terms of adverse interactions. The "calculation" described herein is an electronic calculation, performed preferably automatically by the electronic processor on which the software program is executed.

In short, therefore, the program allows you to perform at least a calculation of a first score for cocktail 100 and a second score for the combination of drugs; the comparison of the first score and the second score allows understanding whether the combination of drugs is, for the subject 200, better than the cocktail 100 in terms at least of reduction of adverse interactions.

In a preferred and non-limiting embodiment, in turn the combination of drugs may comprise over-the-counter drugs and/or medications subject to medical prescription, and/or supplements and/or vitamins and/or phytopharmaceuticals and/or herbs.

Preferably but not limited to, the software program object of the present disclosure is designed to treat a total number D of drugs greater than 5, and therefore to make an optimization on "polytreated" subjects 200 according to the definition given by the WHO.

In the detailed description which follows, reference is made to an exemplary embodiment of the software program described herein, in which increasing scores are indexes of increasingly greater adverse or negative interactions, and in which decreasing scores are indexes of increasingly lower adverse or negative interactions. In this case, the optimization of the drug cocktail for a patient is given by a reduction in the score. Clearly, it is possible to conceive a software program that operates, with the opposite logic, but with the criteria described below, in which the score is calculated in an increasing manner when adverse or negative interactions are reduced. In this second and alternative case, increasing scores are indexes of increasingly lower adverse or negative interactions, while decreasing scores are indexes of increasingly greater adverse or negative interactions.

In particular, the software program described herein considers a list and score of genetic variants that is regularly updated by an international consortium to describe the level of clinical evidence of these variants. This, for example, means that if an enzyme is very slow (inefficient metabolizer) but it is also important in the degradation of a drug (or prodrug activation) there may be variations in the score that may be positive or negative (for example, induction vs. inhibition, extended metabolite vs. inhibition) which may be partially compensated for one another. The Applicant notes that the genetic variants are between 1% and 90% of the ethnic group under examination, and must therefore be taken into due consideration.

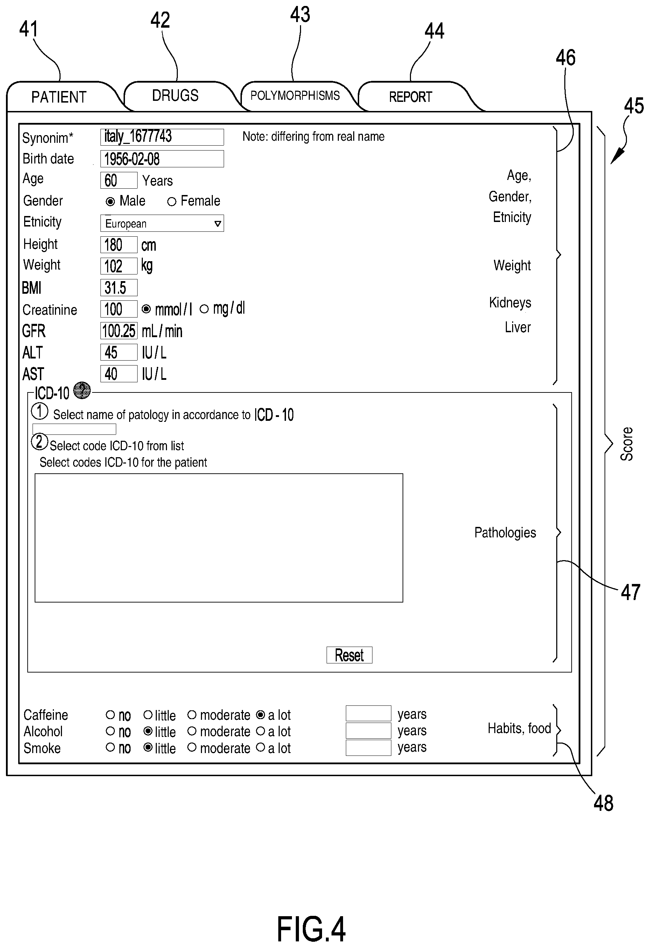

The software program first presents a first screen - shown in figure 4 - in which for a given subject 200 a plurality of patient data is entered by a user, who may be the attending physician or the patient himself. To allow this introduction, the software program preferably but not limited to, presents a first tab 41 for entering patient data.

These patient data are divided into three categories. A first category is related to personal parameters and/or blood values 46, a second category is related to current or past diseases 47 of the patient and a third category includes habit data 48 for caffeine and/or alcohol and/or smoke. The introduction of habit data 48 for caffeine and/or alcohol and/or smoke allows the score to be determined more precisely: in fact, caffeine and/or alcohol and/or smoke interact with the functionality of certain enzymes, gradually changing their functionality with the increase in intake doses. Although the mere mention of the usual caffeine and/or alcohol and/or smoking intake may be sufficient to determine which, among the various enzymes involved in the metabolization of a drug, are affected by the habit of subject 200, the precise identification of intake doses allows obtaining greater calculation reliability and accuracy.

In particular, among the personal parameters and/or blood values 46 that can be entered, at least part of the following data, and preferably all the following data are required: date of birth, age, gender, ethnicity, height, weight, body mass index, creatinine (expressed in mmol/l or mg/dl), GRF, bilirubin, gamma-glutamyl transpeptidase (GGT), alkaline phosphatase (or ALP), ALT, AST. This latest data is indicative of kidney and liver efficiency. Also, data related to intestinal function of the subject 200 may be present.

Preferably, although not limited to, the personal parameters may also comprise data relating to the lifestyle of said subject 200 and in particular the number of weekly hours devoted to exercise and/or exposure to the work environment and/or diet data including at least the indication of an omnivorous, vegetarian or vegan diet and/or the number and distribution of meals during the day, and/or comorbidity data.

The diseases 47 are selected according to the ICD-10 standard, so that the software program can be easily used even in extremely remote geographical areas while maintaining consistency of analysis of the type of disease. A first field allows the introduction of the name of the disease, for example asthma. According to the name of the disease entered, a drop-down menu appears below the first field and allows you to select one or more of the results deriving from the previously mentioned name. It is possible to reset the type of disease selected using a special button.

Preferably, but not limited to, among the habit data 48, entering all three data relating to caffeine, alcohol, and smoke is required. Due to this aspect, the analysis of drug interactions and the nest drug cocktail 100 for the patient is optimized. In particular, the habit data 48 may be expressed on a frequency scale for example comprising a first value corresponding to absence of intake, a second value corresponding to a poor intake quantity, a third value corresponding to a moderate intake quantity and a fourth value corresponding to a high intake quantity. Preferably, for each of the caffeine, alcohol and smoking data, the software program disclosed herein requires indicating the number of years of intake.

Preferably, although not limited to, the calculation of said score includes the analysis of clinical laboratory data, among which there are data belonging to the following list:
- hormonal profile;
- intestinal permeability;
- hematological profile;
- coagulation profile;
- functional biological markers of endogenous and exogenous homeostasis.
These clinical laboratory data are therefore factors that contribute to the determination of said score, and possibly alternate the value of the score calculated before they were taken into account.

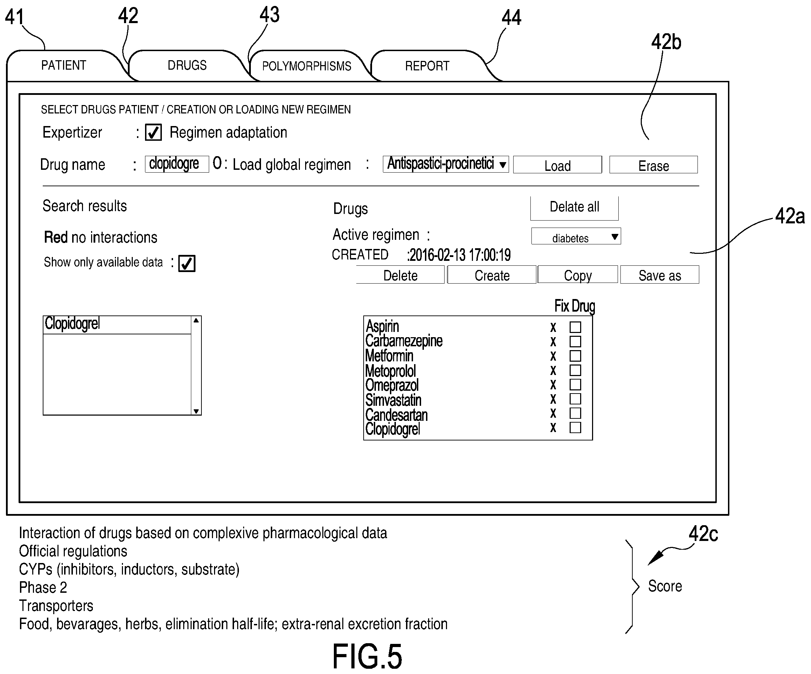

Figure 5 illustrates a screen of the software program disclosed herein in which a second tab 42 relates to the drugs taken by the patient. From this tab, the software program allows access to a database of drugs of a known type and/or commercial name.

In particular, in the second tab 42 it is possible to identify an upper portion 42b in which there is a field in which the user can enter a name of a drug, for example omeprazole or clopidogrel, or select a list of drugs from a list for classification according to global regimes. According to the present invention by "regime" or "global regime", or "pharmacological class", it is meant the particular pharmaceutical category of a plurality of drugs, for example the category of antispasmodics and/or prokinetics, or antihypertensives, or anti-inflammatories or psychotropic drugs. There may be a first button to load the global regime and a second button to cancel the global regime.

In a lower portion 42a of the tab 42, the results of the search are displayed, for example by entering the partial name of a drug in the aforementioned field. In this lower portion 42a there is in particular a list of drugs associated with the name of the drug entered in the aforementioned field and/or associated with the selected global regime, and buttons may be present to cancel the drug regime, to create a regime, to copy the contents thereof or to save the regime as a global regime. In particular, the names of the drug molecule are identified in the drug list and, preferably, not the commercial names. Due to this aspect, it is easier for the user to search each drug more precisely and effectively; in fact the commercial names of the drugs may vary from country to country, while the molecule typically maintains the name in a substantially unaltered manner, or with modest linguistic variations, for all the countries. For each drug shown in the list, there is a checkbox that allows you to select the specific drug of interest.

The selection of drugs according to the second tab allows contributing to the identification of the score to be assigned to the drug cocktail 100. From the selection of the drugs taken by the patient, a known drug cocktail 100 is therefore defined. The software program, on the basis of such a cocktail 100, accesses a previously established database of drugs and extracts interaction data between drugs obtained from total pharmaceutical data comprising at least one, and preferably all, the following data:
- official provisions,
- Phase I and II metabolic enzymes (inhibitor drugs, inducing drugs, substrate drugs),
- transporters,
- molecular targets,
- clearance half-life, extra renal secreted fraction.

Preferably, moreover, not only is the extra renal secreted fraction taken into account, but also data regarding the concentration of drugs in the various biological liquids are considered, according to the therapeutic monitoring of the drugs.

These data are processed according to food and drink intake data (habit data 48) previously entered in the first tab 41, in order to contribute to the definition of the aforementioned score.

The software program also includes a third tab, identified with the reference numeral 43. This third tab includes data relating to polymorphisms.

A first portion of the aforementioned third tab 43 relates to enzymes. Here it is possible to select the enzyme phase (for example, and not limited to, transporters), the main type, the allele and the genotype.

A second portion of the third tab 43 allows defining data relating to the single nucleotide polymorphisms (SNP) for the specific patient whose data are examined. In this second portion:
- a first column defines the protein (enzyme, transporter or target) interacting with the drug (e.g. ABCB1, CYP2C19),
- a second column defines the allele (for example, for the enzyme CYP2C19, allele 10 or 2),
- a third column defines the identification number of the polymorphism (rsid),
- a fourth column defines the nucleotide variation (for example 1236>T, for the enzyme ABCB1),
- a fifth column identifies the activity associated with the specific allele, and includes at least a first "reduced" alternative or a second "increased" alternative,
- a sixth column and a seventh column identify a homozygous or heterozygous characteristic, respectively.

In particular, therefore, the software program is configured to calculate if at least one enzyme and, more preferably, a plurality of enzymes and even more preferably all the enzymes involved in the metabolization of the drugs (D) of the cocktail 100, if each e-th enzyme exhibits reduced or increased activity in relation to the effect provided by the drugs of the cocktail.

A third portion of the third tab 43 has data extracted from an overall database of single nucleotide polymorphisms (SNP). This database, for the purposes of the present disclosure, may be the previously mentioned drug database. This third portion also has:
- a first column which defines the protein (enzyme, transporter or target) interacting with the drug (e.g. ABCB1, CYP2C19),
- a second column which defines the allele (for example, for the enzyme CYP2C19, allele 10 or 2),
- a third column which defines the identification number of the polymorphism (rsid),
- a fourth column which defines the nucleotide variation (for example 1236>T, for the enzyme ABCB1),
- a fifth column which identifies the activity associated with the specific allele, and includes at least a first "reduced" alternative or a second "increased" alternative,
- a sixth column and a seventh column which identify a homozygous or heterozygous characteristic, respectively,
- an eighth column in which, for each polymorphism, therefore for each row, there is an add button.

If the user clicks on this button, the corresponding polymorphism, and its values according to the columns from the second to the sixth, are loaded as data for the specific patient under examination. A scroll bar may be present on the third portion of the third tab, if the total number of database rows cannot be entirely displayed on the screen.

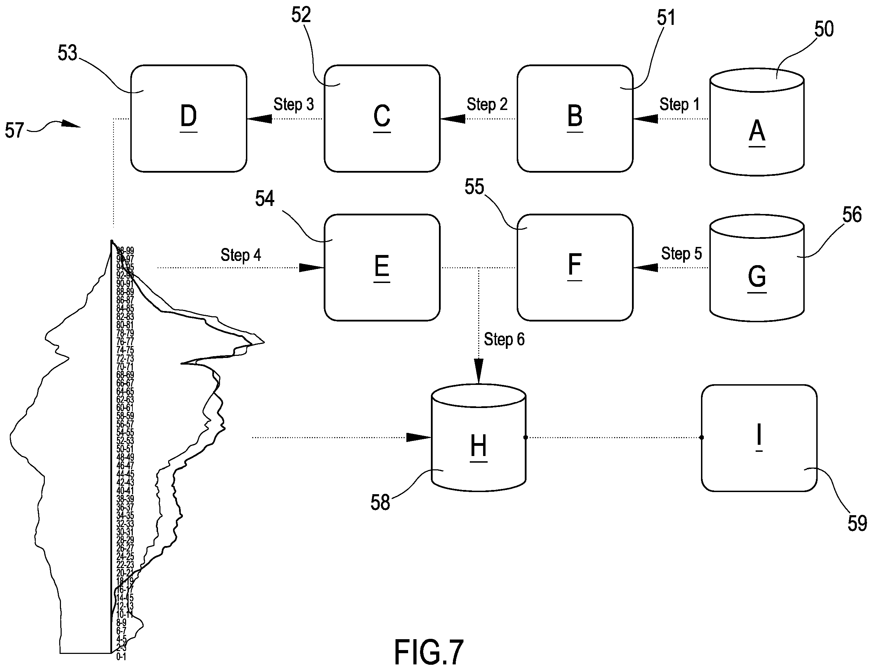

Figure 7 illustrates a scheme of distribution of the scores (with negative logic, therefore higher scores corresponding to adverse or negative interactions) for age group distribution in relation to an analysis process used for implementing the software program according to the present disclosure.

In particular, a first step of the analysis of drug interactions and/or side effects that occurred (identified with reference numeral 50) consisted in the analysis of letters produced by doctors, with a sample of 2 million entities. A second step B (identified with the reference numeral 51), of letters concerning discarded medicaments followed such a first step, with a sample of 500,000 entities. Subsequently, the interactions between drugs and/or side effects have been evaluated by means of anonymous letters, in which it is not clear what the source of the communication is.

A third step derives from the analysis (identified with reference numeral 54) of medication summaries, diagnosis according to gender and age; through these steps it was possible to obtain a diagram 57 which correlates adverse effects to age groups and from which it is observed that as the average age of the subject 200 increases, the number of adverse interactions increases, only to decrease in old age, substantially above the limit of 77-80 years.

Subsequently, also due to a fourth step (a step identified by the reference numeral 54), through the names of the downloaded drugs, a database 58 of discharge letters was defined, which was created also taking into account any differences in the commercial names and ATC (Anatomical Therapeutic Classification system) codes. For the purposes of the present disclosure, the database 58 may be integrated into the previously mentioned drug database. Such differences and ATC codes were taken from a source of 10,000 data which in turn is generated (fifth step, identified by reference numeral 56) from data from pharmaceutical companies.

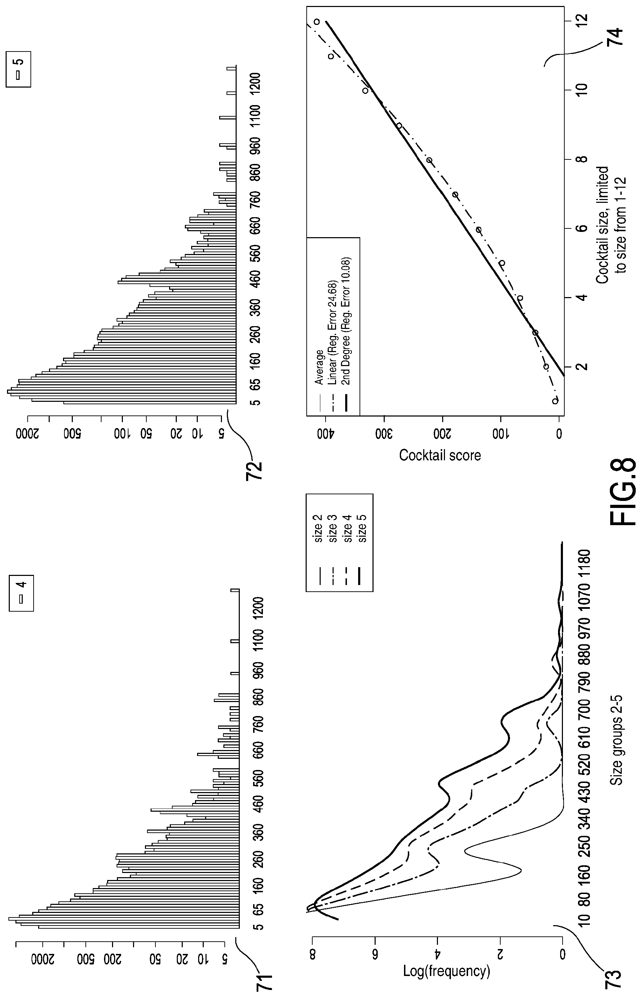

Figure 8 illustrates, in the upper left, a first graph in which the score for a cocktail 100 of four drugs is present on the abscissa and the number of occurrences of the determined score identified in the abscissa is present on the ordinate; figure 7, top right, illustrates a second graph which, similarly to the first graph, shows the score for a cocktail 100 of five drugs on the abscissa and shows the number of occurrences of the determined score identified on the abscissa on the ordinate.

Lower left, figure 8 illustrates a third graph which on the abscissa indicates the score for a drug cocktail 100 defined according to the selected curve and on the ordinate indicates the number of occurrences of the determined score on a logarithmic scale; in particular, the graph in the lower left of figure 8 represents values for cocktail 100 of two, three, four and five drugs.

At the bottom right, figure 8 illustrates a graph in which the number of drugs in the cocktail 100 is present on the abscissa, and the average score for a cocktail of a certain number of drugs is presented on the ordinate. It is clear that cocktails 100 with a small number of drugs exhibit significantly lower scores than the scores of cocktails 100 with a large number of drugs, a sign that adverse interactions and/or side effects on the individual increase on average more and more with the increase in the number of drugs in cocktail 100. Moreover, the points identified on said graph show a non-linear curve with increasing derivative, a sign that the increase in the score and therefore the risk of adverse interactions and/or side effects is more than proportional to the increase in the number of drugs in cocktail 100.

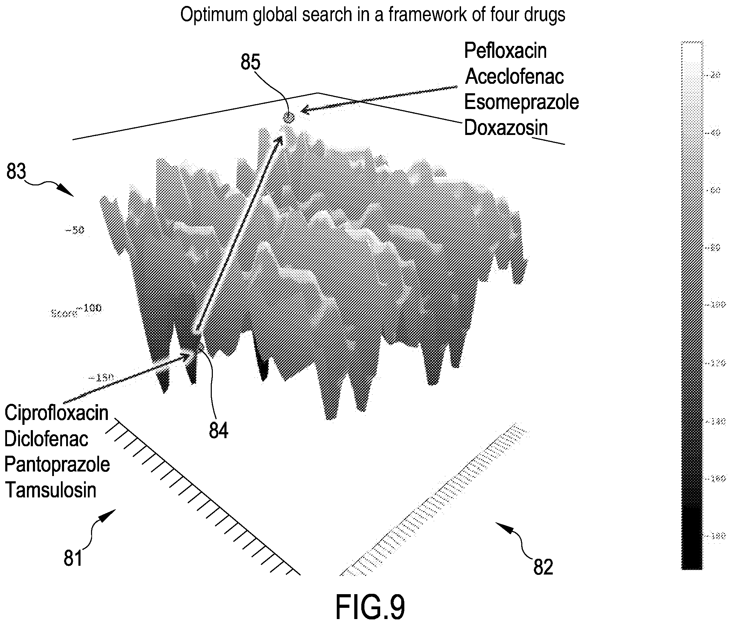

Figure 9 illustrates a diagram in which in relation to a first factor identified with the reference numeral 81, and a second factor identified with the reference numeral 82, a three-dimensional curve of scores is identified for a cocktail 100 of 4 drugs. In the diagram in figure 9, the lower (better) score is represented by the highest points of the curve, while higher (worse) scores are represented by lower points. From a value close to 150, corresponding to a cocktail of four drugs comprising Ciprofloxacin, Diclofenac, Pantoprazole, Tamsulosin, an improvement was obtained, bringing the score to a value significantly lower than 50, with a cocktail of four drugs comprising Pefloxacin, Aceclofenac, Esomeprazole and Doxazosin. However, the four drugs contribute to treating the same diseases already identified for the others.

In the software program, the user appropriately enters the list of drugs that are part of the drug cocktail 100, in particular by selecting the commercial name of the drug and/or its active ingredient. Optionally, the dose taken for each drug of said drug cocktail 100 and/or the duration of administration of each drug of said drug cocktail 100 are also entered. The "cocktail" 100, according to the present invention therefore represents the set of drugs currently taken by the subject 200.

When the commercial name of a drug is entered by the user, the software program is configured to electronically connect to a database of drugs, and to automatically search for one or more active ingredients or non-commercial names of drugs associated with said commercial name.

These data, together with the patient's own data, are stored, at least temporarily, in a file associated with the specific subject 200.

The software program in particular is conceived to allow the execution of an electronic calculation step in which, following the entry of the data of the subject 200 as indicated above, a score is calculated as a function of a summation comprising:
- a first plurality of sum factors, wherein each of said factors takes into account drug interactions (TIₑ, Iₑ), and of a corrective factor (M_{c,e}) extracted from a matrix (M) of corrective factors, each including an interaction factor between a predefined drug, in particular the c-th drug and a predefined protein (enzyme, transporter or target) in particular the e-th protein;
- a second plurality of sum factors, wherein each factor takes into account alarm and/or warning signs (S_{d}, W_{d}), of a correction factor deriving from the action of prodrugs (Pro_{d}), and of a physiological correction factor (Pharm_{d}), related to physiological data of said subject 200.

The software program is further configured to carry out an optimization step of said score, wherein in said step the value of said score corresponding to the minimum possible adverse interaction is sought. The sum factors are calculated taking into account at least the enzymes involved in the metabolization of the drugs taken by the subject 200, and in order to guarantee the best possible uniformity of calculation, the aforementioned factors are calculated electronically taking into account the non-commercial names of the drugs and/or the active ingredients.

As already mentioned, the software program disclosed herein may operate with positive logic or negative logic. For this reason, we have chosen to define the optimal value as "the value of said score corresponding to the minimum possible adverse interaction" as a minimum value of a curve defined on at least two dimensions and/or of a multidimensional curve when increasing scores are indicative of increasing adverse interactions, and is a maximum value of a curve defined on at least two dimensions and/or of a multidimensional curve when decreasing scores are indicative of increasing adverse interactions. Preferably, although not limited to, such valid minimum or maximum values are respectively and/or ideally absolute minimum and/or absolute maximum values.

In particular, the software program disclosed herein is specifically designed to perform the calculation of the following formula: ${\sum }_{e = 1}^{E} {\sum }_{c = S , Inh , Ind} \left({TI}_{e}\times {I}_{c , e}\times {M}_{c , e}\right) + {\sum }_{d = 1}^{D} {S}_{d} + {W}_{d} + {Pro}_{d} + {Pharm}_{d}$ wherein:
- E represents the total number of proteins, and in particular the total number of metabolizing enzymes, including cytochrome P450;
- e represents the e-th enzyme;
- D represents the total number of drugs forming part of the cocktail 100 taken by the patient;
- d represents the d-th drug;
- TIₑ represents, for each enzyme e, the total number of interactions between the drugs forming part of the cocktail 100 taken by the patient;
- I_{c,e} represents, for each enzyme e, the total number of type "c" interactions between the drugs forming part of the cocktail 100 taken by the patient;
- M_{c,e} represents the matrix correction factor for each type "c" interaction, on the enzymatic activity a(e);
- S_{d} represents, for the d-th drug, a stop sign;
- W_{d} represents, for the d-th drug, an alert sign;
- Pro_{d} represents, for the d-th drug, a factor of modification of the prodrug score;
- Pharm_{d} represents, for the d-th drug, an additional corrective term based on physiological factors;
- "c" type interactions are interactions between substrate drugs, inhibitors, inducers and targets.

In relation to the above formulation, "first plurality of sum factors" means the following portion of the above formula: ${\sum }_{e = 1}^{E} {\sum }_{c = S , Inh , Ind} \left({TI}_{e}\times {I}_{c , e}\times {M}_{c , a}\right)$

In relation to the above formulation, "second plurality of sum factors" means the following portion of the above formula: ${\sum }_{d = 1}^{D} {S}_{d} + {W}_{d} + {Pro}_{d} + {Pharm}_{d}$

In particular, the number E identifies those enzymes that are able to make the drugs more hydrophilic, by excretion, by adding a component such as for example and not limited to, glucuronic acid.

As already observed, "M" denotes a matrix of corrective factors identified by the notation M_{c,e}, therefore a corrective factor that takes into account, and/or is calculated in relation to, the c-th drug of the drug cocktail 100 taken by the subject 200 under examination, and to the e-th enzyme examined.

In general, the lower each corrective factor value is, the better it is, since the severity of the interaction is gradually reduced. In general, also, the lower the total number of enzymes that actively contribute (therefore, with positive additions) to define the above summation, the better.

The factors S_{d}, W_{d} mentioned above comprise data and/or legal rules retrieved from illustrative leaflets and/or medical information intended for professionals of the health sector, and in particular relate to fatal cases due to interactions between drugs and/or due to combinations between drugs. The Applicant has observed that since the fatal interaction between drugs and/or the fatal combination of drugs represent the worst possible negative event related to drug intake, this result being rare must be considered with particular severity in defining the score.

For his reason, said alarm and/or warning factors S_{d}, W_{d} contribute as a factor with priority score in said second plurality of sum factors, and/or for each drug d of said cocktail 100 of drugs, the score relative to the alarm and/or warning factor S_{d}, W_{d}, relative to said drug, is greater and/or preponderant with respect to the score relative to the correction factor deriving from prodrugs Pro_{d}, and/or the physiological correction factor Pharm_{d}. Therefore, in a preferred and non-limiting embodiment of the software program disclosed herein, if an alarm and/or warning factor S_{d}, W_{d} with a score different from a predetermined acceptability value (preferably equal to zero) is identified for a d-th drug of said cocktail 100, the software program described herein is configured to emit an alarm signal, for example a sound signal, and/or to display on video, for example and not limited to in the form of a stop signal and/or halt, an appropriate warning sign.

The Applicant underlines how important it is to carry out a check of the alarm and/or warning factors for all the drugs in the drug cocktail proposed to the subject 200, since the substitution of a drug with another drug of the same regime could have serious and even fatal effects. Therefore, such a check is automatically repeated by the software program disclosed herein even if the user selects alternative drugs to a d-th drug in the cocktail 100 proposed to the subject 200 or used by the subject 200. It can therefore be generally asserted that the minimums (or maximums, depending on the program operating logic) of the multidimensional curve described above can only be found for those drug cocktail 100 in which there are no alarm or warning factors.

Having observed the specific function of the prodrugs in the role of metabolization, the corrective factor deriving from the action of prodrugs (Pro_{d}) has greater weight than the physiological corrective factor (Pharm_{d}), relative to physiological data of said subject 200; in the case of prodrugs, in fact, the adverse effects may be of greater importance.

The software program disclosed herein allows searching for, and if possible providing, an alternative, and therefore different, combination of drugs with respect to cocktail 100, being able to select one or more alternative drugs to at least one and preferably each d-th drug in cocktail 100. This allows a search for the optimization of the score, proposing a better combination of drugs than the one contained in the cocktail 100. This research is technically performed by searching in the drug database queried by the software those drugs whose active ingredient is used to treat the same disease as the d-th drug and/or those drugs that are part of the same general regime.

The Applicant has conceived two particular non-limiting embodiments of the software program disclosed herein, which are presented hereinafter. A first non-limiting embodiment of the software program allows testing all the medicines of each general drug regimen present in the drug cocktail 100, with all the combinations of all the other drugs, in order to autonomously search for the optimal score value and being able to define the entire range of scores for the cocktail 100 under consideration. A second embodiment of the software program disclosed herein otherwise allows the user to interact more deeply; in particular, the user can define a sub-sector or subset of drugs of cocktail 100 considered as essential; such a sub-sector or subset is for convenience of description herein referred to as "S". When the sub-sector or subset S is defined, the user can, for the remaining drugs of the cocktail 100 and in particular for each general regime, manually search for an alternative solution among the pharmaceutical alternatives proposed by the program.

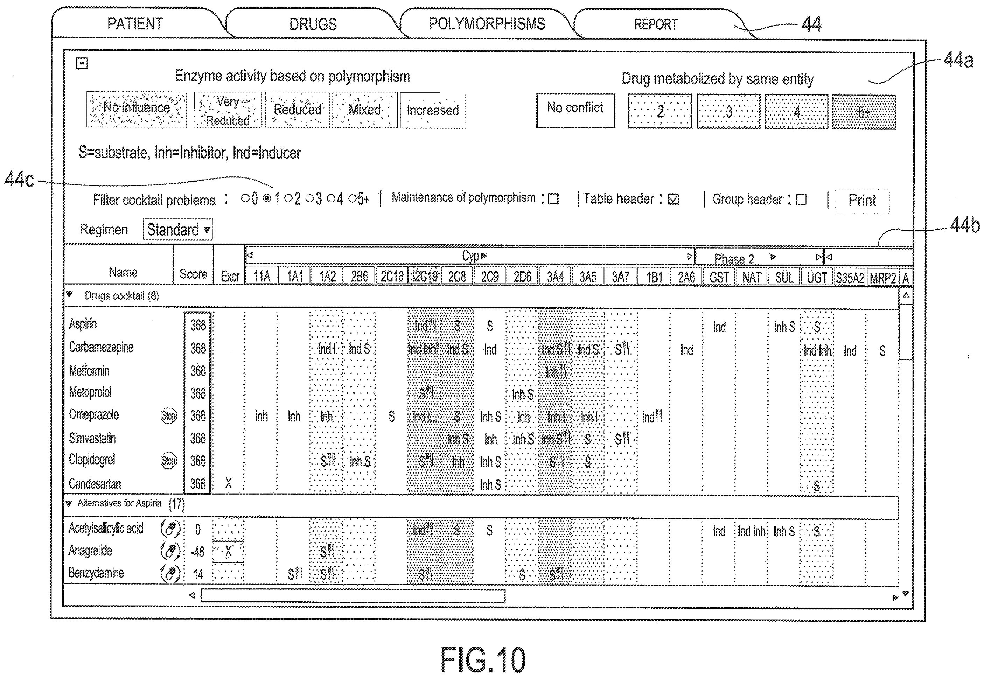

Figure 10 shows a fourth tab 44 of the software program disclosed herein. This fourth tab is a report tab, i.e. which shows a result comprising said score calculated for the predetermined patient when the data have been correctly entered in the first tab 41, in the second tab 42, in the third tab 43 and in the fourth tab 44.

A first portion of the fourth tab 44 helps to identify the functionality of the protein based on the polymorphism, allowing to identify through graphic differentiation the absence of influence with respect to a very reduced activity, or reduced, or mixed, or increased. Still in the first portion of the fourth tab 44, it is possible to identify by means of graphic differentiation the number of drugs metabolized or interacting with the same entity (for example, and not limited to, 2 or 3 or 4 or 5 or more drugs).

A second portion of the fourth tab allows filtering the results obtained in relation to the number of problems 44c for the drug cocktail 100. In particular, in figure 10 a screen is identified that allows selecting among zero, or one, or two, or three, or four, or five or more problems being able to also select or not the maintenance of the polymorphism between the displayed data.

Below the second portion, a third portion comprises a table in which the drugs of the cocktail 100 taken by the patient are highlighted in a first column, preferably the leftmost one. Each row corresponds to a specific drug, for example Aspirin or Omeprazole, among those included in cocktail 100. A second column, juxtaposed to the first, indicates the score for the drug cocktail 100. The score is clearly the same for each row, since it is relative to the entire cocktail 100. A third column, subdivided into several sub-columns, concerns the cytochrome P450, the main responsible for the functioning of drugs within the human body, and each sub-column concerns a specific enzymatic isoform. In particular, the non-limiting representation in figure 10 shows sub-columns for the following enzymatic isoforms: 11A, 1A1, 1A2, 2B6, 2C18, 2C19, 2C8, 2C9, 2D6, 3A4, 3A5, 3A7, 1B1, 2A6. A fourth column, subdivided into several sub-columns, concerns phase 2 enzymes, responsible for the cellular biotransformation mechanisms. The sub-columns of the fourth column relate to the enzyme GST, the enzyme NAT, the enzyme SUL, and the enzyme UGT.

For each cell there are the following indications:
- Inh: represents an inhibitory activity of the drug on the function of the protein (enzyme or transporter);
- Ind: represents an activity of induction of the drug on the function of the protein (enzyme or transporter);
- S: represents the quality of the drug to be substrate of the activity of the protein (enzyme or transporter);
- Ind S: represents the property of the drug to be both a substrate of the activity of the protein and to exert an induction activity on the function of the protein (enzyme or transporter);
- Inh S: represents the property of the drug to be both a substrate of the activity of the protein and to exert an inhibitory activity on the function of the protein (enzyme or transporter);
- fork symbol: indications of variation in the diet to favor a reduction in the score so far determined.

It should be noted that some lines, at the first column, may show a STOP symbol. This symbol, just like the symbol of the hand present in some other rows of the table, as described above, indicates an adverse interaction with a fatal outcome.

A fourth portion of the fourth tab 44 may show a list of alternative drugs for one or more drugs present in the above table. This list is presented in tabular form and incorporates the columns mentioned above. For example, in the non-limiting representation in figure 10, acetylsalicylic acid, benzydamine and anagrelide are indicated as substitutes for Aspirin. For each substitute, the second column shows a score of either zero or negative (preceded by a "-" sign) or positive. This score represents the relative decrease or increase on the score reported above if the drug shown in the intermediate table is replaced by its substitute indicated in the fourth portion. For example, in the non-limiting representation in figure 10, the substitution of salicylic acid with aspirin does not entail any advantage, but the replacement of aspirin with anagrelide would lead to a reduction of the score equal to 48.

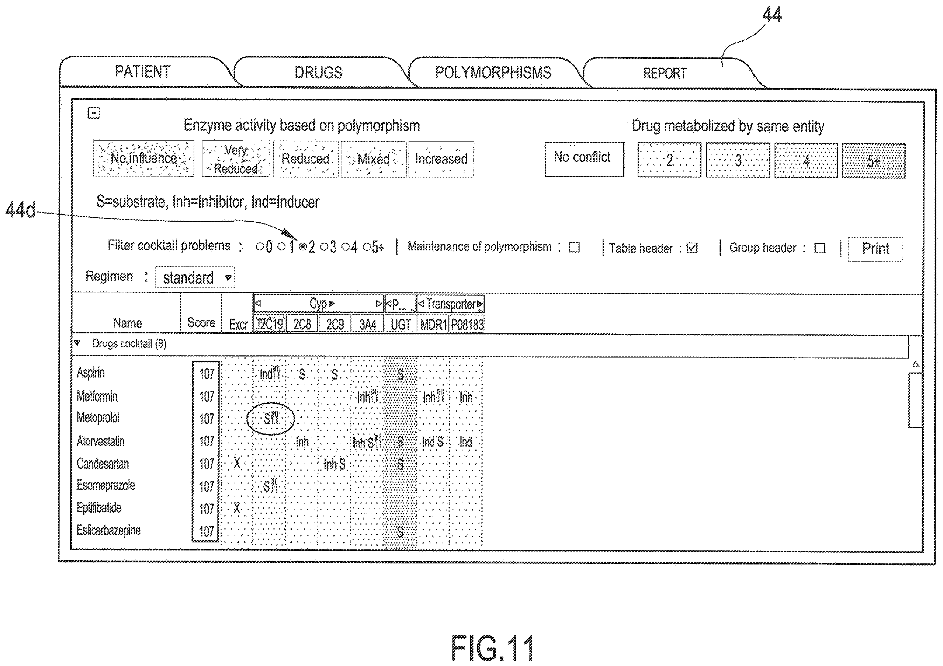

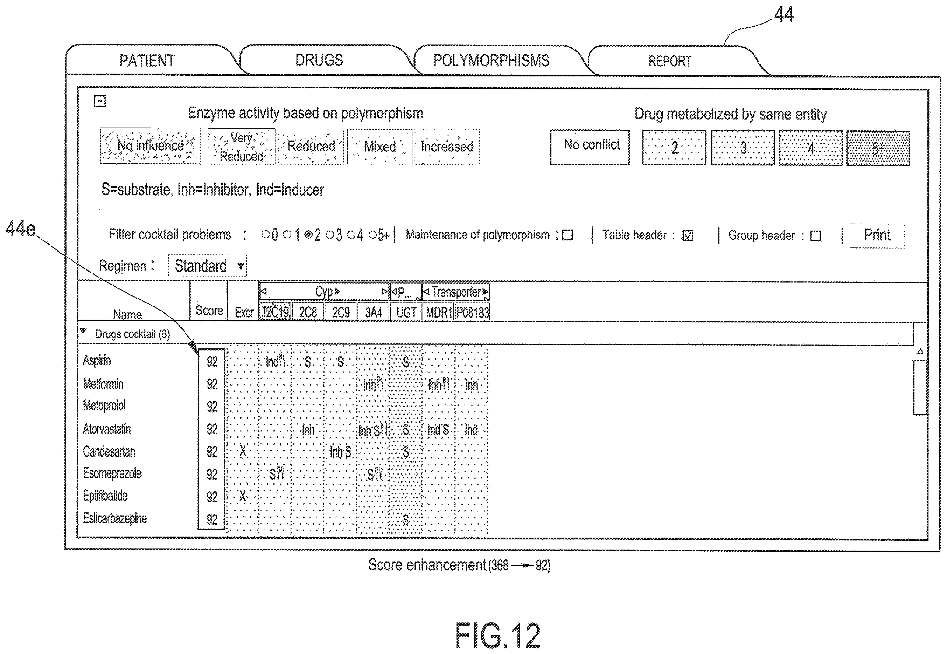

Figure 11 and figure 12 illustrate variations in the score obtainable through an adjustment or variation of the diet taken by a particular individual. In particular, the drug cocktail 100 in both figures includes aspirin, metformin, metoprolol, atorvastatin, candesartan, esomeprazole, eptifibatide, eslicarbazepine, but through the variation of the diet indicated in figure 11, the new score 44e fell to the value of 92 compared to the starting value of 107.

The advantages of the software program described herein are clear in light of the above description. It allows the user, on the basis of a plurality of patient data and knowledge of a drug cocktail 100 currently taken, to identify whether there are adaptations for the cocktail 100 capable of reducing the adverse effects and/or the interactions that such drugs as a whole have on the individual's body, proposing a combination of drugs alternative to the cocktail; this is carried out in a particularly efficient manner since it takes into account the specific genotype of the patient under examination. The use of the software program object of the present disclosure often allows reducing the cost of purchase of the drugs by the patient, and increases the quality of life, often reducing the time required for the treatment of the disease. Through the software program object of the present disclosure it is possible to search for an optimization of the cocktail 100, that is, to search for a combination of alternative drugs that exhibit less drawbacks and negative interactions, within a few minutes.

The genomic data are preferably but not limited to, updated at predetermined time intervals, preferably on a monthly basis, in particular with an update of the genomic variants according to the acquisition of adequate scientific knowledge. These genomic data also include somatic genomic and microbial flora processing data from various body sources including the intestinal tract, skin, oral and/or synovial cavity, biopsies.

The computer program is typically stored on a non-transient memory support, and comprises portions of software code which when executed by a data processing unit lead to the execution of the procedures described above.

Such software program can be written in any one programming language of known type. The computer may be a single unit or on the contrary may be formed by several units; if there are two or more of these, such computers may be connected together by means of a data connection such that their calculation powers are shared in any manner; the same computers can therefore be installed in positions that are even geographically different from each other. The computer program may also be configured to be executed through a web browser.

The data processing unit can be a processor of general purpose type, especially configured through said software or firmware program in order to perform one or more parts of the method identified in the present invention, or be an ASIC or dedicated processor, specifically programmed for performing at least part of the operations of the method or process of the present invention.

The non-transient memory support for containing the aforementioned portion of software or firmware program may be internal or external to the processor itself, possibly also external to the electronic processor, and specifically, it may be a memory geographically located remotely with respect to the electronic processor. The memory support may also be physically divided, in the form of a "cloud".

While in the above detailed description reference has been made to a software program designed to operate on an electronic computer, it is clear that it is possible to equivalently conceive the software and computer program set as a single system, configured to manage the interaction between drugs.

The invention is not limited to the embodiments shown in the drawings. Therefore, it should be understood that where features mentioned in the claims are followed by reference numerals or signs, such signs are included only for the purpose of improving the intelligibility of the claims and do not in any way limit the scope of protection provided by the claims themselves.

## Claims

1. Method of management and calculation of the interaction between drugs, said method comprising:
- a step of introducing, for at least one subject (200), a plurality of electronic data (101) relative to at least:
- personal parameters and/or blood values (46),
- current or past diseases (47) of said subject (200);
- habit data (48) relating caffeine and/or alcohol and/or smoke;
wherein as a result of said introduction step these data are at least temporarily stored in a memory support;
- a step of identification of the cocktail (100) of drugs taken by said subject (200);
- a step of definition of a number (D) of drugs taken by said subject (200), wherein said number (D) corresponds to a number of drugs of said cocktail (100), and of a number of enzymes (E) involved in the metabolization of the drugs taken by said subject (200);
the method being **characterized in** further comprising:
- a calculation step, wherein, following the introduction of said data, a score is calculated through the following formula: 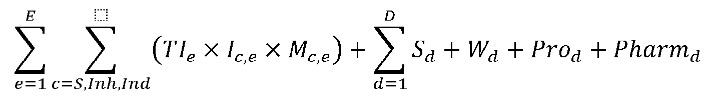 wherein:
- E represents the total number of proteins, and in particular the total number of metabolizing enzymes, including cytochrome P450;
- e represents the e-th enzyme;
- D represents the total number of drugs forming part of the cocktail 100 taken by the patient;
- d represents the d-th drug;
- TIₑ represents, for each enzyme e, the total number of interactions between the drugs forming part of the cocktail 100 taken by the patient;
- I_{c,e} represents, for each enzyme e, the total number of type "c" interactions between the drugs forming part of the cocktail 100 taken by the patient;
- M_{c,e} represents the matrix correction factor for each type "c" interaction, on the enzymatic activity a(e);
- S_{d} represents, for the d-th drug, a stop sign;
- W_{d} represents, for the d-th drug, an alert sign;
- Pro_{d} represents, for the d-th drug, a factor of modification of the prodrug score;
- Pharm_{d} represents, for the d-th drug, an additional corrective term based on physiological factors;
- "c" type interactions are interactions between substrate drugs, inhibitors, inducers and targets;
- a computer implemented optimization research step of said score, wherein in said step a value of said score corresponding to a minimum of adverse interaction between the drugs of said cocktail (100) is sought,
wherein said computer implemented optimization research step of said score comprises the research and electronic proposition of at least one drug as an alternative to one or more drugs of said cocktail (100), said electronic proposition being calculated by automatically searching, for a d-th drug of said cocktail (100), a drug whose active ingredient is of the same pharmacological class as the d-th drug and/or serves for treating the same disease.

2. Method according to claim 1, wherein said introduction of electronic data (101) comprises the introduction of said data (101) into an electronic computer, and comprises the saving, in particular at least temporarily, of said electronic data (101), and wherein the cocktail (100) of drugs includes over-the-counter drugs and/or drugs subject to medical prescription, and/or supplements and/or vitamins and/or phytopharmaceuticals and/or herbs.

3. Method according to one or more of the preceding claims, wherein the computer implemented optimization research step of said score comprises the proposition of a combination of drugs distinct with respect to said cocktail (100), in particular wherein said combination of drugs comprises a plurality of drugs of which at least one is a substitute drug, different from the drugs of said cocktail (100) and/or which contributes or makes said combination of drugs different from said cocktail (100), and/or wherein said combination of drugs comprises a smaller number of drugs than the number of drugs forming part of said cocktail (100).

4. Method according to claim 3, wherein:
- said computer implemented optimization research step of the score is an electronic optimization research step automatically performed by a software program and/or electronically and/or automatically performed by an electronic computer, and/or
- wherein said computer implemented optimization research step of said score comprises the electronic calculation of a first score based on the drugs of said cocktail (100) and an electronic calculation of a second score based on the drugs of said combination of drugs,
said substitute drug contributing to optimizing said score, and/or contributes to producing a smaller adverse interaction between the drugs of said drug combination compared to the adverse interaction that occurs between the drugs of said cocktail,
and/or when said substitute drug is substituted for a corresponding drug in said cocktail, it leads to a reduction of the adverse interaction between the drugs of said cocktail.

5. Method according to one or more of the preceding claims, wherein:
- at least said calculation step, and/or said score optimization step, are performed by electronic access to a drug database (304) and/or following access from a drug database (304), in particular from which interaction data and/or data related to side effects are taken;
- said calculation step take into account the genotype of said subject (200);
- the summation in said formula is performed for a predefined number (E) of enzymes (c), optionally for at least a part and/or for a plurality of enzymes, preferably all enzymes, involved in the metabolization of at least one drug, and preferably of all the drugs, of said cocktail (100).

6. Method according to one or more of the preceding claims, wherein, following the introduction of said habit data (48), there is a step of electronic search of data for inhibiting and/or affecting enzymes in association with said habit data, in particular enzymes affected or inhibited by the use of alcohol and/or caffeine and/or smoke;
said calculation step being performed by a correction of the score induced and/or generated by said enzyme inhibition and/or affection data;
and wherein said score is calculated on each drug (d) and/or on the total number (D) of drugs of said cocktail (100) of drugs taken by the subject (200).

7. Method according to one or more of the preceding claims, wherein, in the calculation step, a first plurality of sum factors is represented by the following formula: ${\sum }_{e = 1}^{E} {\sum }_{c = S , Inh , Ind} \left({TI}_{e}\times {I}_{c , e}\times {M}_{c , a}\right)$ and a second plurality of sum factors is represented by the following formula: ${\sum }_{d = 1}^{D} {S}_{d} + {W}_{d} + {Pro}_{d} + {Pharm}_{d} .$

8. Method according to the preceding claim, wherein at least the calculation of said first plurality of sum factors and of the second plurality of sum factors is performed in relation to non-commercial names and/or active ingredients of drugs stored in said drug database (304), and/or is performed taking into account the interaction data and/or related to side effects contained in said drug database (304), in particular concerning at least one drug (d) of the cocktail (100).

9. Method according to claim 7 or 8, wherein the step of identifying said cocktail (100) of drugs comprises identifying and storing the commercial and/or non-commercial name and/or the active ingredient of one or more drugs, optionally further comprising the storage of the doses taken for each drug of said cocktail (100) of drugs and/or the duration of administration of each drug of said cocktail (100) of drugs;
wherein said first plurality of sum factors is calculated on at least a predefined number of enzymes (c), optionally on all the enzymes (E), active in the metabolization of drugs, and is calculated taking into account at least the cytochrome P450;
and wherein the interaction factors between drugs comprise the total number of interactions (TIₑ) of the drugs forming part of said cocktail (100).

10. Method according to one or more of the preceding claims 7-9, wherein in said first plurality of sum factors, said corrective factor (Mc,e) extracted from the matrix (M) of corrective factors is multiplied by the factors of drug interactions (TIe, Ie),
wherein said drug interaction factors comprise the total number (TIe) of interactions between all the drugs taken by said subject (200) and all the enzymes (e) and the number of type C interactions (Ie) for all the drugs taken of said cocktail (100) and all the enzymes (e),
and wherein the total number (TIe) of interactions between all the drugs taken by said subject (200) and all the enzymes (e) and the number of type C interactions (Ie) for all the drugs taken by said subject (200) and all the enzymes (e) are multiplied together to contribute to define said factor of the first plurality of sum factors,
and wherein the total number (TIe) of interactions among all the drugs taken by said subject (200) and all the enzymes (e) and the number of C-type interactions (I) for all the drugs taken by said subject (200) and all the enzymes (e), is multiplied by said corrective factor (Mc,e) deriving from said matrix (M) of values, each comprising an interaction factor between a predefined drug and a predefined enzyme.

11. Method according to one or more of the preceding claims, wherein the personal parameters and/or blood values (46) comprise at least part of the following data, and preferably all the following data: date of birth, age, gender, ethnicity, height, weight, body mass index, creatinine, GRF, bilirubin, gamma-glutamyl transpeptidase (GGT), alkaline phosphatase (or ALP), ALT, AST,
and wherein there is a step of selecting said drug as an alternative to one or more drugs of said drug cocktail (100), in particular to lead to the creation of said combination of drugs, following which the calculation step is preferably performed again.

12. Method according to claim 3 and claim 11, wherein as a result of said selection step, said drug as an alternative to one or more drugs of said cocktail (100) is added to and/or becomes part of said composition of drugs alternative to said cocktail (100), becoming said substitute drug.

13. Method according to one or more of the preceding claims, comprising an electronic presentation step, on a user interface, of at least one tab (43) relating to single nucleotide polymorphisms (SNP) for said subject (200), in which data are presented relating to one or more enzymes (e), and optionally, for each enzyme (e) of said plurality of enzymes, there are data relating to the allele of the enzyme, to a nucleotide variation thereof and/or to a change in the activity associated with the enzyme or of the enzyme.

14. Method according to claim 13, comprising an electronic calculation step of the variation of the activity of one or more enzymes (e) of said plurality of enzymes, said variation being calculated based on, and/or being generated by and or because of one or more drugs (d) of said cocktail (100).

15. Method according to one or more of the preceding claims, wherein the calculation step of said score includes a prior analysis and/or consideration of clinical laboratory data, said clinical laboratory data comprising at least one of the data of the following list:
- hormonal profile;
- intestinal permeability;
- hematological profile;
- coagulation profile;
- functional biological markers of endogenous and exogenous homeostasis,
and wherein said clinical laboratory data contribute to the determination and/or alteration of said score.

16. Software program comprising software code portions which, when executed by a data processing unit, cause the execution of the steps according to one or more of the preceding claims.

17. System (300) for managing interactions between drugs comprising at least a data processing unit and a memory (304), on which a software program for the management of drug interactions is loaded, said data processing unit being configured to execute said software program which, when executed, executes the method according to one or more of claims 1 to 15.

18. System (300) according to claim 17, comprising at least one access interface to allow simultaneous access to the use of said software program, optionally by a plurality of users simultaneously.

## Patentansprüche

1. Verfahren zur Verwaltung und Berechnung der Wechselwirkung zwischen Arzneimitteln, wobei das Verfahren umfasst:
- einen Schritt des Einführens, für wenigstens ein Individuum (200), einer Mehrzahl elektronischer Daten (101) bezüglich wenigstens:
- persönlicher Parameter und/oder Blutwerte (46),
- aktueller oder vergangener Erkrankungen (47) des Individuums (200);
- Gewohnheitsdaten (48) betreffend Koffein und/oder Alkohol und/oder Rauchen;
wobei als ein Ergebnis des Einführungsschritts diese Daten wenigstens vorübergehend in einem Speichermedium gespeichert werden;
- einen Schritt einer Identifikation des Cocktails (100) von Arzneimitteln, welche von dem Individuum (200) eingenommen werden;
- einen Schritt einer Definition einer Anzahl (D) von Arzneimitteln, welche von dem Individuum (200) eingenommen werden, wobei die Anzahl (D) einer Anzahl von Arzneimitteln des Cocktails (100) entspricht, sowie einer Anzahl von Enzymen (E), welche an der Metabolisierung von Arzneimitteln beteiligt sind, welche von dem Individuum (200) eingenommen werden;
wobei das Verfahren **dadurch gekennzeichnet ist, dass** es ferner umfasst:
- einen Berechnungsschritt, wobei, nach dem Einführen der Daten, ein Score durch die folgende Formel berechnet wird: 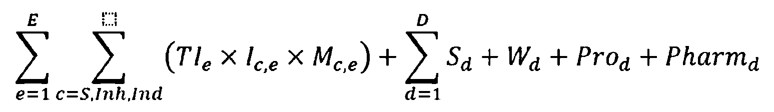 wobei:
- E die Gesamtanzahl von Proteinen und insbesondere die Gesamtanzahl von metabolisierenden Enzymen, einschließlich Cytochrom P450, darstellt;
- e das e-te Enzym darstellt;
- D die Gesamtanzahl von Arzneimitteln darstellt, welche Teil des Cocktails (100) bilden, welcher vom Patienten eingenommen wird;
- d das d-te Arzneimittel darstellt;
- TIₑ, für jedes Enzym e, die Gesamtanzahl von Wechselwirkungen zwischen den Arzneimitteln darstellt, welche Teil des Cocktails (100) bilden, welcher vom Patienten eingenommen wird;
- I_{c,e}, für jedes Enzym e, die Gesamtanzahl von Wechselwirkungen vom Typ "c" zwischen den Arzneimitteln darstellt, welche Teil des Cocktails (100) bilden, welcher vom Patienten eingenommen wird;
- M_{c,e} den Matrixkorrekturfaktor für jede Wechselwirkung vom Typ "c" auf die enzymatische Aktivität a(e) darstellt;
- S_{d}, für das d-te Arzneimittel, ein Stoppzeichen darstellt;
- W_{d}, für das d-te Arzneimittel, ein Alarmzeichen darstellt;
- Pro_{d}, für das d-te Arzneimittel, einen Modifikationsfaktor des Prodrug-Scores darstellt;
- Pharm_{d}, für das d-te Arzneimittel, einen zusätzlichen Korrekturterm auf Grundlage physiologischer Faktoren darstellt;
- Wechselwirkungen vom Typ "c" Wechselwirkungen zwischen Substrat-Arzneimitteln, Inhibitoren, Induktoren und Targets sind;
- einen computerimplementierten Optimierungsrechercheschritt des Scores, wobei in dem Schritt ein Wert des Scores gesucht wird, welcher einem Minimum unerwünschter Wechselwirkungen zwischen den Arzneimitteln des Cocktails (100) entspricht,
wobei der computerimplementierte Optimierungsrechercheschritt des Scores die Recherche und den elektronischen Vorschlag wenigstens eines Arzneimittels als eine Alternative zu einem oder mehreren Arzneimitteln des Cocktails (100) umfasst, wobei der elektronische Vorschlag berechnet wird, indem für ein d-tes Arzneimittel des Cocktails (100) automatisch ein Arzneimittel gesucht wird, dessen Wirkstoff derselben pharmakologischen Klasse angehört wie das d-te Arzneimittel und/oder zur Behandlung derselben Erkrankung dient.

2. Verfahren nach Anspruch 1, wobei das Einführen elektronischer Daten (101) das Einführen der Daten (101) in einen elektronischen Computer umfasst und das Speichern, insbesondere wenigstens vorübergehend, der elektronischen Daten (101) umfasst, und wobei der Cocktail (100) von Arzneimitteln rezeptfreie Arzneimittel und/oder verschreibungspflichtige Arzneimittel und/oder Nahrungsergänzungsmittel und/oder Vitamine und/oder Phytopharmaka und/oder Kräuter umfasst.

3. Verfahren nach einem oder mehreren der vorhergehenden Ansprüche, wobei der computerimplementierte Optimierungsrechercheschritt des Scores den Vorschlag einer Arzneimittelkombination umfasst, welche sich von dem Cocktail (100) unterscheidet, insbesondere wobei die Arzneimittelkombination eine Mehrzahl von Arzneimitteln umfasst, von denen wenigstens eines ein Ersatzarzneimittel ist, welches sich von den Arzneimitteln des Cocktails (100) unterscheidet und/oder welches dazu beiträgt oder bewirkt, dass sich die Arzneimittelkombination von dem Cocktail (100) unterscheidet, und/oder wobei die Arzneimittelkombination eine geringere Anzahl von Arzneimitteln umfasst als die Anzahl der Arzneimittel, welche Teil des Cocktails (100) sind.

4. Verfahren nach Anspruch 3, wobei:
- der computerimplementierte Optimierungsrechercheschritt des Scores ein elektronischer Optimierungsrechercheschritt ist, welcher automatisch durch ein Softwareprogramm und/oder elektronisch und/oder automatisch durch einen elektronischen Computer durchgeführt wird, und/oder
- wobei der computerimplementierte Optimierungsrechercheschritt des Scores die elektronische Berechnung eines ersten Scores auf Grundlage der Arzneimittel des Cocktails (100) und eine elektronische Berechnung eines zweiten Scores auf Grundlage der Arzneimittel der Arzneimittelkombination umfasst,
wobei das Ersatzarzneimittel dazu beiträgt, den Score zu optimieren, und/oder dazu beiträgt, eine geringere unerwünschte Wechselwirkung zwischen den Arzneimitteln der Arzneimittelkombination im Vergleich zu der unerwünschten Wechselwirkung zu erzeugen, welche zwischen den Arzneimitteln des Cocktails auftritt, und/oder wenn das Ersatzarzneimittel für ein entsprechendes Arzneimittel des Cocktails ersetzt wird, zu einer Verringerung der unerwünschten Wechselwirkung zwischen den Arzneimitteln des Cocktails führt.

5. Verfahren nach einem oder mehreren der vorhergehenden Ansprüche, wobei:
- wenigstens der Berechnungsschritt und/oder der Score-Optimierungsschritt durch elektronischen Zugriff auf eine Arzneimitteldatenbank (304) und/oder nach Zugriff auf eine Arzneimitteldatenbank (304) durchgeführt werden, insbesondere aus welcher Wechselwirkungsdaten und/oder Daten betreffend Nebenwirkungen entnommen werden;
- der Berechnungsschritt den Genotyp des Individuums (200) berücksichtigt;
- die Summation in der Formel für eine vorbestimmte Anzahl (E) von Enzymen (c) durchgeführt wird, optional für wenigstens einen Teil und/oder für eine Mehrzahl von Enzymen, vorzugsweise alle Enzyme, welche an der Metabolisierung wenigstens eines Arzneimittels, und vorzugsweise aller Arzneimittel, des Cocktails (100) beteiligt sind.

6. Verfahren nach einem oder mehreren der vorhergehenden Ansprüche, wobei, nach dem Einführen der Gewohnheitsdaten (48), ein Schritt der elektronischen Recherche von Daten zu inhibierenden und/oder beeinflussenden Enzymen in Verbindung mit den Gewohnheitsdaten erfolgt, insbesondere Enzyme, welche durch den Konsum von Alkohol und/oder Koffein und/oder Rauchen beeinflusst oder gehemmt werden;
wobei der Berechnungsschritt durch eine Korrektur des Scores durchgeführt wird, welche durch die Enzymhemmungs- und/oder -beeinflussungsdaten induziert und/oder erzeugt wird; und wobei der Score für jedes Arzneimittel (d) und/oder für die Gesamtanzahl (D) der Arzneimittel des Cocktails (100) von Arzneimitteln, welche von dem Individuum (200) eingenommen werden, berechnet wird.

7. Verfahren nach einem oder mehreren der vorhergehenden Ansprüche, wobei im Berechnungsschritt eine erste Mehrzahl von Summenfaktoren durch die folgende Formel dargestellt wird: 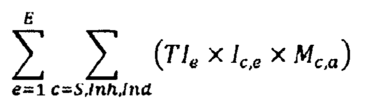 und eine zweite Mehrzahl von Summenfaktoren durch die folgende Formel dargestellt wird:

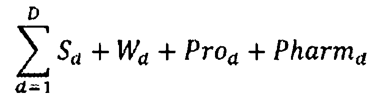

8. Verfahren nach dem vorhergehenden Anspruch, wobei wenigstens die Berechnung der ersten Mehrzahl von Summenfaktoren und der zweiten Mehrzahl von Summenfaktoren in Bezug auf Nicht-Handelsnamen und/oder Wirkstoffe von Arzneimitteln durchgeführt wird, welche in der Arzneimitteldatenbank (304) gespeichert sind, und/oder unter Berücksichtigung der Wechselwirkungsdaten und/oder der Daten betreffend Nebenwirkungen durchgeführt wird, welche in der Arzneimitteldatenbank (304) enthalten sind, insbesondere hinsichtlich wenigstens eines Arzneimittels (d) des Cocktails (100).

9. Verfahren nach Anspruch 7 oder 8, wobei der Schritt des Identifizierens des Cocktails (100) von Arzneimitteln Identifizieren und Speichern des Handelsnamens und/oder Nicht-Handelsnamens und/oder des Wirkstoffs eines oder mehrerer Arzneimittel umfasst, optional ferner umfassend das Speichern der Dosen, welche für jedes Arzneimittel des Cocktails (100) von Arzneimitteln eingenommen werden, und/oder der Dauer der Verabreichung jedes Arzneimittels des Cocktails (100) von Arzneimitteln;
wobei die erste Mehrzahl von Summenfaktoren für wenigstens eine vorbestimmte Anzahl von Enzymen (c), optional für alle Enzyme (E), welche bei der Metabolisierung von Arzneimitteln aktiv sind, berechnet wird und unter Berücksichtigung wenigstens des Cytochrom P450 berechnet wird;
und wobei die Wechselwirkungsfaktoren zwischen Arzneimitteln die Gesamtanzahl der Wechselwirkungen (Tle) der Arzneimittel umfassen, welche Teil des Cocktails (100) bilden.

10. Verfahren nach einem oder mehreren der vorhergehenden Ansprüche 7-9, wobei in der ersten Mehrzahl von Summenfaktoren der Korrekturfaktor (Mc,e), welcher aus der Matrix (M) von Korrekturfaktoren extrahiert wird, mit den Faktoren der Arzneimittelwechselwirkung (Tle, le) multipliziert wird, wobei die Arzneimittelwechselwirkungsfaktoren die Gesamtanzahl (Tle) der Wechselwirkungen zwischen allen Arzneimitteln, welche von dem Individuum (200) eingenommen werden, und allen Enzymen (e) sowie die Anzahl der Wechselwirkungen vom Typ C (Ie) für alle eingenommenen Arzneimittel des Cocktails (100) und allen Enzymen (e) umfassen,
und wobei die Gesamtanzahl (Tle) von Wechselwirkungen zwischen allen Arzneimitteln, welche von dem Individuum (200) eingenommen werden, und allen Enzymen (e) sowie die Anzahl der Wechselwirkungen vom Typ C (Ic,e) für alle Arzneimittel, welche von dem Individuum (200) eingenommen werden, und allen Enzymen (e) miteinander multipliziert werden, um dazu beizutragen, den Faktor der ersten Mehrzahl von Summenfaktoren zu definieren,
und wobei die Gesamtanzahl (Tle) von Wechselwirkungen zwischen allen Arzneimitteln, welche von dem Individuum (200) eingenommen werden, und allen Enzymen (e) sowie die Anzahl der Wechselwirkungen vom Typ C (I) für alle Arzneimittel, welche von dem Individuum (200) eingenommen werden, und allen Enzymen (e) mit dem Korrekturfaktor (Mc,e) multipliziert werden, welcher aus der Matrix (M) von Werten abgeleitet wird, wobei jeder davon einen Wechselwirkungsfaktor zwischen einem vorbestimmten Arzneimittel und einem vorbestimmten Enzym umfasst.

11. Verfahren nach einem oder mehreren der vorhergehenden Ansprüche, wobei die persönlichen Parameter und/oder Blutwerte (46) wenigstens einen Teil der folgenden Daten, und vorzugsweise alle folgenden Daten, umfassen: Geburtsdatum, Alter, Geschlecht, Ethnizität, Körpergröße, Körpergewicht, Body-Mass-Index, Kreatinin, GRF, Bilirubin, Gamma-Glutamyltransferase (GGT), alkalische Phosphatase (oder ALP), ALT, AST,
und wobei ein Schritt des Auswählens des Arzneimittels als eine Alternative zu einem oder mehreren Arzneimitteln des Arzneimittelcocktails (100) vorgesehen ist, insbesondere um die Erstellung der Arzneimittelkombination herbeizuführen, woraufhin der Berechnungsschritt vorzugsweise erneut durchgeführt wird.

12. Verfahren nach Anspruch 3 und Anspruch 11, wobei als ein Ergebnis des Auswahlschritts das Arzneimittel als eine Alternative zu einem oder mehreren Arzneimitteln des Cocktails (100) der Arzneimittelzusammensetzung als Alternative zu dem Cocktail (100) hinzugefügt wird und/oder Teil davon wird und zum Ersatzarzneimittel wird.

13. Verfahren nach einem oder mehreren der vorhergehenden Ansprüche, umfassend einen elektronischen Präsentationsschritt, auf einer Benutzeroberfläche, wenigstens einer Registerkarte (43) betreffend Einzelnukleotid-Polymorphismen (SNP) für das Individuum (200), in welcher Daten betreffend ein oder mehrere Enzyme (e) präsentiert werden, und optional, für jedes Enzym (e) der Mehrzahl von Enzymen, Daten betreffend das Allel des Enzyms, eine Nukleotidvariation davon und/oder eine mit dem Enzym oder dem Enzym assoziierte Aktivitätsänderung vorliegen.

14. Verfahren nach Anspruch 13, umfassend einen elektronischen Berechnungsschritt der Aktivitätsvariation eines oder mehrerer Enzyme (e) der Mehrzahl von Enzymen, wobei die Variation auf Grundlage von und/oder erzeugt durch und/oder aufgrund eines oder mehrerer Arzneimittel (d) des Cocktails (100) berechnet wird.

15. Verfahren nach einem oder mehreren der vorhergehenden Ansprüche, wobei der Berechnungsschritt des Scores eine vorherige Analyse und/oder Berücksichtigung klinischer Labordaten umfasst, wobei die klinischen Labordaten wenigstens eines der Daten der folgenden Liste umfassen:
- hormonelles Profil;
- intestinale Permeabilität;
- hämatologisches Profil;
- Gerinnungsprofil;
- funktionelle biologische Marker endogener und exogener Homöostase,
und wobei die klinischen Labordaten zur Bestimmung und/oder Veränderung des Scores beitragen.

16. Softwareprogramm, umfassend Softwarecodeabschnitte, welche, wenn sie durch eine Datenverarbeitungseinheit ausgeführt werden, die Ausführung der Schritte nach einem oder mehreren der vorhergehenden Ansprüche bewirken.

17. System (300) zum Verwalten von Wechselwirkungen zwischen Arzneimitteln, umfassend wenigstens eine Datenverarbeitungseinheit und einen Speicher (304), auf welchem ein Softwareprogramm für die Verwaltung von Arzneimittelwechselwirkungen geladen ist, wobei die Datenverarbeitungseinheit dazu eingerichtet ist, das Softwareprogramm auszuführen, welches, wenn es ausgeführt wird, das Verfahren nach einem oder mehreren der Ansprüche 1 bis 15 ausführt.

18. System (300) nach Anspruch 17, umfassend wenigstens eine Zugriffsschnittstelle, um einen gleichzeitigen Zugriff auf die Nutzung des Softwareprogramms zu ermöglichen, optional durch eine Mehrzahl von Nutzern gleichzeitig.

## Revendications

1. Procédé de gestion et de calcul de l'interaction entre des médicaments, ledit procédé comprenant :
- une étape d'introduction, pour au moins un sujet (200), d'une pluralité de données électroniques (101) concernant au moins :
- des paramètres individuels et/ou des valeurs sanguines (46),
- des maladies (47) présentes ou passées dudit sujet (200) ;
- des données d'habitude (48) concernant la caféine et/ou l'alcool et/ou la prise de tabac ;
dans lequel en résultat de ladite étape d'introduction ces données sont au moins temporairement stockées dans un support type mémoire ;
- une étape d'identification du cocktail (100) de médicaments pris par ledit sujet (200) ;
- une étape de définition d'un nombre (D) de médicaments pris par ledit sujet (200), dans lequel ledit nombre (D) correspond à un nombre de médicaments dudit cocktail (100), et d'un nombre d'enzymes (E) impliquées dans la métabolisation des médicaments pris par ledit sujet (200) ;
le procédé étant **caractérisé en ce qu'**il comprend en outre :
- une étape de calcul, dans laquelle, suite à l'introduction desdites données, une note est calculée à travers la formule suivante : 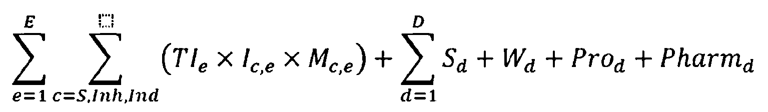 dans laquelle
- E représente le nombre total de protéines, et en particulier le nombre total d'enzymes de métabolisation, incluant le cytochrome P450 ;
- e représente la n-ième enzyme ;
- D représente le nombre total de médicaments formant une partie du cocktail 100 pris par le patient ;
- d représente le d-ième médicament ;
- TIₑ représente, pour chaque enzyme e, le nombre total d'interactions entre les médicaments formant une partie du cocktail 100 pris par le patient ;
- I_{c,e} représente, pour chaque enzyme e, le nombre total d'interactions de type « c » entre les médicaments formant une partie du cocktail 100 pris par le patient ;
- M_{c,e} représente le facteur de correction de la matrice pour chaque interaction de type « c », sur l'activité enzymatique a(e) ;
- S_{d} représente, pour le d-ième médicament, un signe d'arrêt ;
- W_{d} représente, pour le d-ième médicament, un signe d'alerte ;
- Pro_{d} représente, pour le d-ième médicament, un facteur de modification de la note de promédicament ;
- Pharm_{d} représente, pour le d-ième médicament, un terme de correction additionnel basé sur des facteurs physiologiques ;
- les interactions de type « c » sont des interactions entre les médicaments formant substrat, les inhibiteurs, les inducteurs et les cibles ;
- une étape de recherche d'optimisation mise en œuvre par ordinateur de ladite note, dans laquelle dans ladite étape, une valeur de ladite note correspondant à un minimum d'interaction défavorable entre les médicaments dudit cocktail (100) est recherché,
dans lequel ladite étape de recherche d'optimisation de ladite note mise en œuvre par ordinateur comprend la recherche et la proposition électronique d'au moins un médicament comme alternative à l'un ou à plusieurs médicaments dudit cocktail (100), ladite proposition électronique étant calculée en recherchant automatiquement, pour un d-ième médicament dudit cocktail (100), un médicament dont l'ingrédient actif est de la même classe pharmacologique que le d-ième médicament et/ou qui sert au traitement de la même maladie.

2. Procédé selon la revendication 1, dans lequel ladite introduction de données électroniques (101) comprend l'introduction desdites données (101) dans un ordinateur électronique, et comprend la sauvegarde, en particulier au moins temporairement, desdites données électroniques (101), et dans lequel le cocktail (100) de médicaments inclut des médicaments en vente libre et/ou des médicaments soumis à une prescription médicale, et/ou des compléments et/ou des vitamines et/ou des agents phytopharmaceutiques et/ou des herbes.

3. Procédé selon l'une ou plusieurs des revendications précédentes, dans lequel l'étape de recherche d'optimisation mise en œuvre par ordinateur de ladite note comprend la proposition d'une combinaison de médicaments distincts par rapport audit cocktail (100), en particulier dans lequel ladite combinaison de médicaments comprend une pluralité de médicaments dont au moins l'un est un médicament substitut, différent des médicaments dudit cocktail (100) et/ou qui contribue ou qui rend ladite combinaison de médicaments différente dudit cocktail (100), et/ou dans lequel ladite combinaison de médicaments comprend un nombre plus petit de médicaments que le nombre de médicaments formant une partie dudit cocktail (100).

4. Procédé selon la revendication 3, dans lequel :
- ladite étape de recherche d'optimisation mise en œuvre par ordinateur de la note est une étape de recherche d'optimisation électronique automatiquement exécutée par un progiciel et/ou électroniquement et/ou automatiquement exécutée par un ordinateur électronique, et/ou
- dans lequel ladite étape de recherche d'optimisation mise en œuvre par ordinateur de ladite note comprend le calcul électronique d'une première note basé sur les médicaments dudit cocktail (100) et d'un calcul électronique d'une seconde note basé sur les médicaments de ladite combinaison de médicaments,
ledit médicament substitut contribuant à optimiser ladite note, et/ou contribuant à produire une interaction défavorable plus petite entre les médicaments de ladite combinaison de médicaments comparée à l'interaction défavorable qui se produit entre les médicaments dudit cocktail, et/ou lorsque ledit médicament substitut est substitué par un médicament correspondant dans ledit cocktail, il conduit à une réduction de l'interaction défavorable entre les médicaments dudit cocktail.

5. Procédé selon l'une ou plusieurs des revendications précédentes, dans lequel :
- au moins ladite étape de calcul, et/ou ladite étape d'optimisation de note, sont exécutées par accès électronique à une base de données de médicaments (304) et/ou en suivant un accès depuis une base de données de médicaments (304), en particulier depuis laquelle les données d'interaction et/ou les données liées aux effets secondaires sont prises ;
- ladite étape de calcul prend en compte le génotype dudit sujet (200) ;
- la somme dans ladite formule est exécutée pour un nombre prédéfini (E) d'enzymes (c), éventuellement pour au moins une partie et/ou pour une pluralité d'enzymes, préférablement de toutes les enzymes, impliquées dans la métabolisation d'au moins un médicament, et préférablement de tous les médicaments, dudit cocktail (100).

6. Procédé selon l'une ou plusieurs des revendications précédentes, dans lequel, suite à l'introduction desdites données d'habitude (48), il existe une étape de recherche électronique de données pour inhiber et/ou pour affecter les enzymes en association avec lesdites données d'habitude, en particulier des enzymes affectées ou inhibées par l'utilisation d'alcool et/ou de caféine et/ou de prise de tabac ;
ladite étape de calcul étant exécutée par une correction de la note induite et/ou générée par ladite inhibition d'enzyme et/ou les données d'affection ;
et dans laquelle ladite note est calculée sur chaque médicament (d) et/ou sur le nombre total (D) de médicaments dudit cocktail (100) de médicaments pris par le sujet (200).

7. Procédé selon l'une ou plusieurs des revendications précédentes, dans lequel, dans l'étape de calcul, une première pluralité de facteurs de somme est représentée par la formule suivante : 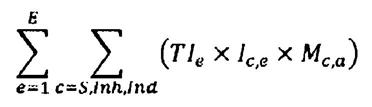 et une seconde pluralité de facteurs de somme est représentée par la formule suivante : ${\sum }_{d = 1}^{D} {S}_{d} + {W}_{d} + {Pro}_{d} + {Pharm}_{d}$

8. Procédé selon la revendication précédente, dans lequel au moins le calcul de ladite première pluralité de facteurs de somme et de la seconde pluralité de facteurs de somme est effectué en relation à des noms non commerciaux et/ou à des ingrédients actifs de médicaments stockés dans ladite base de données de médicaments (304), et/ou est effectué en prenant en compte les données d'interaction et/ou liées aux effets secondaires contenus dans ladite base de données de médicaments (304), en particulier concernant au moins un médicament (d) du cocktail (100).

9. Procédé selon la revendication 7 ou 8, dans lequel l'étape d'identification dudit cocktail (100) de médicaments comprend l'identification et le stockage du nom commercial et/ou non commercial et/ou de l'ingrédient actif d'un ou plusieurs médicaments, éventuellement comprenant en outre le stockage des doses prises pour chaque médicament dudit cocktail (100) de médicaments et/ou de la durée d'administration de chaque médicament dudit cocktail (100) de médicaments ;
dans lequel ladite première pluralité de facteurs de somme est calculée sur au moins un nombre prédéfini d'enzymes (c), éventuellement sur toutes les enzymes (E), actives dans la métabolisation des médicaments, et est calculée en prenant en compte au moins le cytochrome P450 ;
et dans lequel les facteurs d'interaction entre les médicaments comprennent le nombre total d'interactions (TIₑ) des médicaments formant une partie dudit cocktail (100).

10. Procédé selon l'une ou plusieurs des revendications précédentes de 7 à 9, dans lequel dans ladite première pluralité de facteurs de somme, ledit facteur de correction (M_{c,e}) extrait de la matrice (M) des facteurs de correction est multiplié par les facteurs des interactions médicamenteuses (TIₑ, Iₑ),
dans lequel lesdits facteurs d'interaction médicamenteuse comprennent le nombre total (TIₑ) d'interactions entre tous les médicaments pris par ledit sujet (200) et toutes les enzymes (e) et le nombre d'interactions (le) de type C pour tous les médicaments pris dudit cocktail (100) et de toutes les enzymes (e),
et dans lequel le nombre total (TIₑ) d'interactions entre tous les médicaments pris par ledit sujet (200) et toutes les enzymes (e) et le nombre d'interactions (le) de type C pour tous les médicaments pris par ledit sujet (200) et toutes les enzymes (e) sont multipliés conjointement pour contribuer à définir ledit facteur de la première pluralité de facteurs de somme,
et dans lequel le nombre total (TIₑ) d'interactions parmi tous les médicaments pris par ledit sujet (200) et toutes les enzymes (e) et le nombre d'interactions (I) de type C pour tous les médicaments pris par ledit sujet (200) et toutes les enzymes (e), est multiplié par ledit facteur de correction (M_{c,e}) dérivant de ladite matrice (M) de valeurs, comprenant chacune un facteur d'interaction entre un médicament prédéfini et une enzyme prédéfinie.

11. Procédé selon l'une ou plusieurs des revendications précédentes, dans lequel les paramètres individuels et/ou les valeurs sanguines (46) comprennent au moins une partie des données suivantes, et préférablement toutes les données suivantes : date de naissance, âge, sexe, ethnie, taille, poids, indice de masse corporelle, créatinine, GRF, bilirubine, gamma-glutamyl transpeptidase (GGT), alcaline phosphatase (ou ALP), ALT, AST,
et dans lequel il existe une étape de sélection dudit médicament comme alternative à l'un ou à plusieurs médicaments dudit cocktail (100) de médicaments, en particulier pour conduire à la création de ladite combinaison de médicaments, suite à laquelle l'étape de calcul est préférablement à nouveau exécutée.

12. Procédé selon la revendication 3 et la revendication 11, dans lequel en résultat de ladite étape de sélection, ledit médicament comme alternative à l'un ou à plusieurs médicaments dudit cocktail (100) est ajouté à, et/ou devient une partie de, ladite composition de médicaments alternative audit cocktail (100), devenant ledit médicament substitut.

13. Procédé selon l'une ou plusieurs des revendications précédentes, comprenant une étape de présentation électronique, sur une interface utilisateur, d'au moins un onglet (43) concernant des polymorphismes mononucléotidique (PNS) pour ledit sujet (200), dans lequel des données sont présentées concernant une ou plusieurs enzymes (e), et éventuellement, pour chaque enzyme (e) de ladite pluralité d'enzymes, il existe des données liées à l'allèle de l'enzyme, à sa variation nucléotidique et/ou à un changement dans l'activité associée à l'enzyme ou de l'enzyme.

14. Procédé selon la revendication 13, comprenant une étape de calcul électronique de la variation de l'activité d'une ou plusieurs enzymes (e) de ladite pluralité d'enzymes, ladite variation étant calculée sur la base de, et/ou étant générée par, et/ou du fait de, un ou plusieurs médicaments (d) dudit cocktail (100).

15. Procédé selon l'une ou plusieurs des revendications précédentes, dans lequel l'étape de calcul de ladite note inclut une analyse et/ou une prise en considération préalables des données cliniques de laboratoire, lesdites données cliniques de laboratoire comprenant au moins l'un parmi les données de la liste suivante :
- profil hormonal ;
- perméabilité intestinale ;
- profil hématologique ;
- profil de coagulation ;
- marqueurs biologiques fonctionnels de l'homéostasie endogène et exogène,
et dans lequel lesdites données cliniques de laboratoire contribuent à la détermination et/ou à l'altération de ladite note.

16. Progiciel comprenant des portions de code informatique qui, lorsqu'exécutées par une unité de traitement de données, entraînent l'exécution des étapes selon l'une ou plusieurs des revendications précédentes.

17. Système (300) de gestion d'interactions entre des médicaments comprenant au moins une unité de traitement de données et une mémoire (304), sur laquelle un progiciel pour la gestion des interactions médicamenteuses est chargé, ladite unité de traitement de données étant configurée pour exécuter ledit progiciel qui, lorsqu'exécuté, exécute le procédé selon l'une ou plusieurs des revendications 1 à 15.

18. Système (300) selon la revendication 17, comprenant au moins une interface d'accès pour permettre un accès simultané à l'utilisation dudit progiciel, éventuellement simultanément par une pluralité d'utilisateurs.
